# EUROPEAN PATENT APPLICATION

(11) **EP 1 362 929 A2**
(43) Date of publication of application: **19.11.2003**
(21) Application number: 03253110.5
(22) Date of filing: 19.05.2003
(51) Int. Cl.: C12Q 1/68

(54) **Methods for genotyping**

(30) Priority: 17.05.2002 US 319253 P; 04.10.2002 US 264945
(71) Applicant: Affymetrix, Inc., Santa Clara, CA 95051 (US)
(72) Inventor: Dong, Shoulian, San Jose, California 95126 (US); Jones, Keith, Sunnyvale, California 94087 (US); Kennedy, Giulia, San Francisco, California 95116 (US); Liu, Weiwei, Menlo Park, California 94026 (US); Matsuzaki, Hajime, Palo Alto, California 94303 (US); Shapero, Michael, Redwood City, California 94061 (US)
(74) Representative: Williams, Richard Andrew Norman

(57) **Abstract**

Novel methods and kits for analyzing a collection of target sequences in a nucleic acid sample are provided. A reduced complexity sample is generated and then analyzed. A sample is amplified under conditions that enrich for a subset of fragments that includes a collection of target sequences. The invention further provides for analysis of the above sample. Analysis may be by hybridization to an array, which may be specifically designed to interrogate the collection of target sequences for particular characteristics, such as, for example, the presence or absence of one or more polymorphisms.

## Description

### RELATED APPLICATIONS

This application is a continuation-in-part of U.S. Patent Application Nos. 10/264,945 filed October 4, 2002 and 60/319,253 filed May 17, 2002. The entire teachings of the above applications are incorporated herein by reference.

### BACKGROUND OF THE INVENTION

The past years have seen a dynamic change in the ability of science to comprehend vast amounts of data. Pioneering technologies such as nucleic acid arrays allow scientists to delve into the world of genetics in far greater detail than ever before. Exploration of genomic DNA has long been a dream of the scientific community. Held within the complex structures of genomic DNA lies the potential to identify, diagnose, and treat diseases like cancer, Alzheimer disease or alcoholism. Exploitation of genomic information from plants and animals may also provide answers to the world's food distribution problems.

Recent efforts in the scientific community, such as the publication of the draft sequence of the human genome in February 2001, have changed the dream of genome exploration into a reality. Genome-wide assays, however, must contend with the complexity of genomes; the human genome for example is estimated to have a complexity of 3x10⁹ base pairs. However, the clarity and quality of the analysis is, to a large degree, dependent on the quality and complexity of the target nucleic acid interrogated. The present invention provides methods for improving the quality and reducing the complexity of target nucleic acids applied to arrays, thereby improving the quality of the resulting data. Novel methods of sample preparation and sample analysis that reduce complexity may provide for the fast and cost effective exploration of complex samples of nucleic acids from a variety of sources and under a variety of conditions.

### SUMMARY OF THE INVENTION

One embodiment discloses a method of reducing the complexity of a first nucleic acid sample to produce a second nucleic acid sample. The method comprises first selecting a collection of target sequences by a method comprising: identifying fragments that are in a selected size range when a genome is digested with a selected enzyme or enzyme combination; identifying sequences of interest present on the fragments in the selected size range; and selecting as target sequences fragments that are in the selected size range and comprise a sequence of interest. The first nucleic acid sample is fragmented to produce sample fragments and at least one adaptor is ligated to the sample fragments. A second nucleic acid sample is generated by amplifying the fragments. The amplified sample is enriched for a subset of the sample fragments and that subset included a collection of target sequences. In one embodiment the subset of sample fragments is targeted for enrichment by selecting the method of fragmentation.

In one embodiment, amplification of the fragments is by PCR using 20 to 50 cycles. A single primer complementary to the adaptors may be used in some embodiments. In some embodiments two different adaptors are ligated to the fragments and two different primers are used for amplification. In yet another embodiment a single adaptor is used but the adaptor has a double stranded region and single stranded regions. Primers to the single stranded regions are used for amplification. In one embodiment the adaptor sequence comprises a priming site. In another embodiment the adaptor comprises a tag sequence.

In one embodiment, the step of fragmenting the first nucleic acid sample is by digestion with at least one restriction enzyme. The restriction enzyme may, for example, have a 6 base recognition sequence or an 8 base recognition sequence. In some embodiments a type IIs endonuclease is used. In one embodiment fragmenting, ligating and amplifying steps are done in a single tube.

In one embodiment the second nucleic acid sample comprises at least 0.01%, 0.1%, 0.5%, 3%, 10%, 12% or 50% of the first nucleic acid sample. The first nucleic acid sample may be, for example, genomic DNA, DNA, cDNA derived from RNA or cDNA derived from mRNA.

In one embodiment the target sequences are 800, 1000, 1200, 1500, or 2000 base pairs long or less. In one embodiment the subset of sample fragments enriched in the second nucleic acid sample is comprised of fragments that are primarily 2000 or 3000 base pairs long or less.

In one embodiment target sequences contain one or more sequences of interest, such as, for example, sequence variations, such as SNPs. In some embodiments a SNP may be associated with a phenotype, a disease, the efficacy of a drug or with a haplotype.

In another embodiment a method for selecting a collection of target sequences is disclosed. The steps of the method are identifying fragments that are in a selected size range when a genome is digested with a selected enzyme or enzyme combination; identifying sequences of interest present on the fragments in the selected size range; and selecting as target sequences fragments that are in the selected size range and comprise a sequence of interest. In one embodiment a computer system is used for one or more steps of the method. In one embodiment an array is designed to interrogate one or more specific collections of target sequences. In another embodiment a collection of target sequences is disclosed. The collection may be amplified. The collection may also be attached to a solid support.

In another embodiment a method is disclosed for analyzing a collection of target sequences by providing a nucleic acid array; hybridizing the amplified collection of target sequences to the array; generating a hybridization pattern resulting from the hybridization; and analyzing the hybridization pattern. In one embodiment the array is designed to interrogate sequences in the collection of target sequences. In one embodiment the sequences are analyzed to determine if they contain sequence variations, such as SNPs.

In another embodiment, a method for genotyping an individual is disclosed. A collection of target sequences comprising a collection of SNPs is amplified and hybridized to an array comprising probes to interrogate for the presence or absence of different alleles in the collection of SNPs. The hybridization pattern is analyzed to determine which alleles are present for at least one of the SNPs.

In another embodiment a method for screening for DNA sequence variations in a population of individuals is disclosed. Amplified target sequences from each individual are hybridized to an array that interrogates for sequence variation. The hybridization patterns from the arrays are compared to determine the presence or absence of sequence variation in the population of individuals.

In another embodiment kits for genotyping individuals or samples are disclosed. The kit may contain one or more of the following components: buffer, nucleotide triphosphates, a reverse transcriptase, a nuclease, one or more restriction enzymes, two or more adaptors, a ligase, a DNA polymerase, one or more primers and instructions for the use of the kit. In one embodiment the kit contains an array designed to interrogate sequence variation in a collection of target sequences.

In another embodiment a solid support comprising a plurality of probes attached to the solid support is disclosed. The probes may be designed to interrogate sequence variation in a collection of target sequences.

In another embodiment the complexity of a nucleic acid sample is reduced in two steps. In one step the sample is fragmented, ligated to an adaptor and a subset of fragments is amplified. In the other step complexity of the sample is reduced based on a physical or chemical property of the sequence. This step may be removal of repetitive sequences by, for example, incubation with Cot-1 DNA and removal of nucleic acid that is bound to the Cot-1 fraction. The step may be isolation of active chromatin from the first nucleic acid sample wherein the second nucleic acid sample comprises the nucleic acids present in the isolated active chromatin by, for example immunoprecipitation of active chromatin. Antibodies that recognize acetylated histones but not nonacetylated histones may be used, for example. The step may be isolation of one or more individual chromosomes from the first nucleic acid sample by pulsed field gradient gel electrophoresis. Individual chromosomes may be isolated by affinity chromatography using, for example an array or bead as a solid support. A somatic cell hybrid containing a subset of chromosomes from an organism may be used to generate the second nucleic sample. The somatic cell hybrid may contain a reduced subset of a genome, such as a single chromosome or multiple chromosomes or fragments of chromosomes.

The reduced complexity sample may be analyzed, for example, to determine genotypes, using any method known in the art. Methods that may be used to determine the identity of an allele include, but are not limited to, detecting hybridization of a molecular beacon probe, electrical pore analysis, electrical conductance analysis, atomic force microscopy analysis, pyrosequencing, MALDI-TOF mass spectrometry, Surface Enhanced Raman Scattering, current amplitude analysis, use of an eSensor system, and use of electrochemical DNA biosensors.

In another embodiment kits for use with the methods of the invention are disclosed.

### BRIEF DESCRIPTION OF THE DRAWINGS

The foregoing and other objects, features and advantages of the invention will be apparent from the following more particular description of preferred embodiments of the invention, as illustrated in the accompanying drawings in which like reference characters refer to the same parts throughout the different views. The drawings are not necessarily to scale, emphasis instead being placed upon illustrating the principles of the invention.

Figure 1 shows a Venn diagram illustrating how a collection of target sequences may be selected. Potential target sequences are found at the intersection between the set of fragments that contain sequences of interest and the fragments that are in a selected size range. The selected size range is within the set of fragments that are efficiently amplified by PCR under standard conditions.

Figure 2 is a table of the number of SNPs predicted to be found on 400 to 800 base pair fragments when genomic DNA is digested with the restriction enzyme in column 1.

Figure 3 is a flow chart showing design of an array in conjunction with size selection of SNP containing fragments.

Figure 4 is a schematic of a method in which the ends of the adaptors are non-complementary and the fragments are amplified with a primer pair.

### DETAILED DESCRIPTION OF THE INVENTION

A description of preferred embodiments of the invention follows.

While this invention has been particularly shown and described with references to preferred embodiments thereof, it will be understood by those skilled in the art that various changes in form and details may be made therein without departing from the scope of the invention encompassed by the appended claims.

### General

The present invention has many embodiments and relies on many patents, applications and other references for details known to those of the art. Therefore, when a patent, application, or other reference is cited or repeated below, it should be understood that it is incorporated by reference in its entirety for all purposes as well as for the proposition that is recited.

As used in this application, the singular form "a," "an," and "the" include plural references unless the context clearly dictates otherwise. For example, the term "an agent" includes a plurality of agents, including mixtures thereof.

An individual is not limited to a human being but may also be other organisms including but not limited to mammals, plants, bacteria, or cells derived from any of the above.

Throughout this disclosure, various aspects of this invention can be presented in a range format. It should be understood that the description in range format is merely for convenience and brevity and should not be construed as an inflexible limitation on the scope of the invention. Accordingly, the description of a range should be considered to have specifically disclosed all the possible subranges as well as individual numerical values within that range. For example, description of a range such as from 1 to 6 should be considered to have specifically disclosed subranges such as from 1 to 3, from 1 to 4, from 1 to 5, from 2 to 4, from 2 to 6, from 3 to 6 etc., as well as individual numbers within that range, for example, 1, 2, 3, 4, 5, and 6. This applies regardless of the breadth of the range.

The practice of the present invention may employ, unless otherwise indicated, conventional techniques and descriptions of organic chemistry, polymer technology, molecular biology (including recombinant techniques), cell biology, biochemistry, and immunology, which are within the skill of the art. Such conventional techniques include polymer array synthesis, hybridization, ligation, and detection of hybridization using a label. Specific illustrations of suitable techniques can be had by reference to the example herein below. However, other equivalent conventional procedures can, of course, also be used. Such conventional techniques and descriptions can be found in standard laboratory manuals such as *Genome Analysis: A Laboratory Manual Series (Vols. I-IV), Using Antibodies: A Laboratory Manual, Cells: A Laboratory Manual, PCR Primer: A Laboratory Manual,* and Molecular *Cloning: A Laboratory Manual* (all from Cold Spring Harbor Laboratory Press), Stryer, L. (1995) *Biochemistry* (4th Ed.) Freeman, New York, *Gait, "Oligonucleotide Synthesis: A Practical Approach" 1984, IRL Press, London,* Nelson and Cox (2000), *Lehninger, Principles of Biochemistry* 3^{rd} Ed., W.H. Freeman Pub., New York, NY and Berg et al. (2002) *Biochemistry,* 5^{th} Ed., W.H. Freeman Pub., New York, NY, all of which are herein incorporated in their entirety by reference for all purposes.

The present invention can employ solid substrates, including arrays in some embodiments. Methods and techniques applicable to polymer (including protein) array synthesis have been described in U.S.S.N 09/536,841, WO 00/58516, U.S. Patents Nos. 5,143,854, 5,242,974, 5,252,743, 5,324,633, 5,384,261, 5,405,783, 5,424,186, 5,451,683, 5,482,867, 5,491,074, 5,527,681, 5,550,215, 5,571,639, 5,578,832, 5,593,839, 5,599,695, 5,624,711, 5,631,734, 5,795,716, 5,831,070, 5,837,832, 5,856,101, 5,858,659, 5,936,324, 5,968,740, 5,974,164, 5,981,185, 5,981,956, 6,025,601, 6,033,860, 6,040,193, 6,090,555, 6,136,269, 6,269,846 and 6,428,752, in PCT Applications Nos. PCT/US99/00730 (International Publication Number WO 99/36760) and PCT/US01/04285, which are all incorporated herein by reference in their entirety for all purposes.

Patents that describe synthesis techniques in specific embodiments include U.S. Patents Nos. 5,412,087, 6,147,205, 6,262,216, 6,310,189, 5,889,165, and 5,959,098. Nucleic acid arrays are described in many of the above patents, but the same techniques are applied to polypeptide arrays.

The present invention also contemplates many uses for polymers attached to solid substrates. These uses include gene expression monitoring, profiling, library screening, genotyping and diagnostics. Gene expression monitoring, and profiling methods can be shown in U.S. Patents Nos. 5,800,992, 6,013,449, 6,020,135, 6,033,860, 6,040,138, 6,177,248, 6,309,822 and 6,344,316 . Genotyping and uses therefore are shown in USSN 60/319,253, 10/013,598, 10/264,945 and U.S. Patents Nos. 5,856,092, 6,300,063, 5,858,659, 6,284,460, 6,361,947, 6,368,799 and 6,333,179. Other uses are embodied in U.S. Patents Nos. 5,871,928, 5,902,723, 6,045,996, 5,541,061, and 6,197,506.

The present invention also contemplates sample preparation methods in certain embodiments. Prior to or concurrent with genotyping, the genomic sample may be amplified by a variety of mechanisms, some of which may employ PCR. *See,* e.g., *PCR Technology: Principles and Applications for DNA Amplification* (Ed. H.A. Erlich, Freeman Press, NY, NY, 1992); *PCR Protocols: A Guide to Methods and Applications* (Eds. Innis, et al., Academic Press, San Diego, CA, 1990); Mattila et al., *Nucleic Acids Res*. 19, 4967 (1991); Eckert et al., *PCR Methods and Applications* 1, 17 (1991); *PCR* (Eds. McPherson et al., IRL Press, Oxford); and U.S. Patent Nos. 4,683,202, 4,683,195, 4,800,159 4,965,188,and 5,333,675, and each of which is incorporated herein by reference in their entireties for all purposes. The sample may be amplified on the array. See, for example, U.S Patent No 6,300,070 and U.S. patent application 09/513,300, which are incorporated herein by reference.

Other suitable amplification methods include the ligase chain reaction (LCR) (*e.g*.*,* Wu and Wallace, *Genomics* 4, 560 (1989), Landegren et al., *Science* 241, 1077 (1988) and Barringer et al. *Gene* 89:117 (1990)), transcription amplification (Kwoh et al., *Proc. Natl. Acad. Sci. USA* 86, 1173 (1989) and WO88/10315), self-sustained sequence replication (Guatelli et al., *Proc. Nat. Acad. Sci. USA,* 87, 1874 (1990) and WO90/06995), selective amplification of target polynucleotide sequences (U.S. Patent No 6,410,276), consensus sequence primed polymerase chain reaction (CP-PCR) (U.S. Patent No 4,437,975), arbitrarily primed polymerase chain reaction (AP-PCR) (U.S. Patent No 5,413,909, 5,861,245) and nucleic acid based sequence amplification (NABSA). *(See,* US patents nos. 5,409,818, 5,554,517, and 6,063,603, each of which is incorporated herein by reference). Other amplification methods that may be used are described in, U.S. Patent Nos. 5,242,794, 5,494,810, 4,988,617, 6,344,316 and in USSN 09/854,317, each of which is incorporated herein by reference.

Additional methods of sample preparation and techniques for reducing the complexity of a nucleic sample are described in Dong et al., *Genome Research* 11, 1418 (2001), in U.S. Patent No 6,361,947, 6,391,592 and U.S. Patent application Nos. 09/916,135, 09/920,491, 09/910,292, 10/013,598 and 10/264,945.

Methods for conducting polynucleotide hybridization assays have been well developed in the art. Hybridization assay procedures and conditions will vary depending on the application and are selected in accordance with the general binding methods known including those referred to in: Maniatis et al. *Molecular Cloning: A Laboratory Manual* (2^{nd} Ed. Cold Spring Harbor, N.Y, 1989); Berger and Kimmel *Methods in Enzymology,* Vol. 152, *Guide to Molecular Cloning Techniques* (Academic Press, Inc., San Diego, CA, 1987); Young and Davism, *P.N.A.S*, 80: 1194 (1983). Methods and apparatus for carrying out repeated and controlled hybridization reactions have been described in US patent 5,871,928, 5,874,219, 6,045,996 and 6,386,749, 6,391,623 each of which are incorporated herein by reference.

The present invention also contemplates signal detection of hybridization between ligands in certain embodiments. See U.S. Pat. Nos. 5,143,854, 5,578,832; 5,631,734; 5,834,758; 5,936,324; 5,981,956; 6,025,601; 6,141,096; 6,185,030; 6,201,639; 6,218,803; and 6,225,625, in U.S. Patent application 60/435,178 and in PCT Application PCT/U599/06097 (published as WO99/47964), each of which also is hereby incorporated by reference in its entirety for all purposes.

Methods and apparatus for signal detection and processing of intensity data are disclosed in, for example, U.S. Patents Numbers 5,143,854, 5,547,839, 5,578,832, 5,631,734, 5,800,992, 5,834,758; 5,856,092, 5,902,723, 5,936,324, 5,981,956, 6,025,601, 6,090,555, 6,141,096, 6,185,030, 6,201,639; 6,218,803; and 6,225,625, in U.S. Patent application 60/435,178 and in PCT Application PCT/US99/06097 (published as WO99/47964), each of which also is hereby incorporated by reference in its entirety for all purposes.

The practice of the present invention may also employ conventional biology methods, software and systems. Computer software products of the invention typically include computer readable medium having computer-executable instructions for performing the logic steps of the method of the invention. Suitable computer readable medium include floppy disk, CD-ROM/DVD/DVD-ROM, hard-disk drive, flash memory, ROM/RAM, magnetic tapes and etc. The computer executable instructions may be written in a suitable computer language or combination of several languages. Basic computational biology methods are described in, e.g. Setubal and Meidanis et al., *Introduction to Computational Biology Methods* (PWS Publishing Company, Boston, 1997); Salzberg, Searles, Kasif, (Ed.), *Computational Methods in Molecular Biology*, (Elsevier, Amsterdam, 1998); Rashidi and Buehler, *Bioinformatics Basics: Application in Biological Science and Medicine* (CRC Press, London, 2000) and Ouelette and Bzevanis *Bioinformatics: A Practical Guide for Analysis of Gene and Proteins* (Wiley & Sons, Inc., 2^{nd} ed., 2001).

The present invention may also make use of various computer program products and software for a variety of purposes, such as probe design, management of data, analysis, and instrument operation. See, U.S. Patent Nos. 5,593,839, 5,795,716, 5,733,729, 5,974,164, 6,066,454, 6,090,555, 6,185,561, 6,188,783, 6,223,127, 6,229,911 and 6,308,170.

Additionally, the present invention may have embodiments that include methods for providing genetic information over networks such as the Internet as shown in U.S. Patent applications 10/063,559, 60/349,546, 10/423,403, 60/394,574, 60/403,381.

### Definitions

Nucleic acids according to the present invention may include any polymer or oligomer of pyrimidine and purine bases, preferably cytosine (C) , thymine (T), and uracil (U), and adenine (A) and guanine (G), respectively. *See* Albert L. Lehninger, PRINCIPLES OF BIOCHEMISTRY, at 793-800 (Worth Pub. 1982). Indeed, the present invention contemplates any deoxyribonucleotide, ribonucleotide or peptide nucleic acid component, and any chemical variants thereof, such as methylated, hydroxymethylated or glucosylated forms of these bases, and the like. The polymers or oligomers may be heterogeneous or homogeneous in composition, and may be isolated from naturally occurring sources or may be artificially or synthetically produced. In addition, the nucleic acids may be deoxyribonucleic acid (DNA) or ribonucleic acid (RNA), or a mixture thereof, and may exist permanently or transitionally in single-stranded or double-stranded form, including homoduplex, heteroduplex, and hybrid states.

An oligonucleotide or polynucleotide is a nucleic acid ranging from at least 2, preferable at least 8, and more preferably at least 20 nucleotides in length or a compound that specifically hybridizes to a polynucleotide. Polynucleotides of the present invention include sequences of deoxyribonucleic acid (DNA) or ribonucleic acid (RNA), which may be isolated from natural sources, recombinantly produced or artificially synthesized and mimetics thereof. A further example of a polynucleotide of the present invention may be peptide nucleic acid (PNA) in which the constituent bases are joined by peptides bonds rather than phosphodiester linkage, as described in Nielsen et al., *Science* 254:1497-1500 (1991), Nielsen *Curr. Opin. Biotechnol*., 10:71-75 (1999). The invention also encompasses situations in which there is a nontraditional base pairing such as Hoogsteen base pairing which has been identified in certain tRNA molecules and postulated to exist in a triple helix. Polynucleotide and oligonucleotide are used interchangeably in this application.

An array is an intentionally created collection of molecules which can be prepared either synthetically or biosynthetically. The molecules in the array can be identical or different from each other. The array can assume a variety of formats, *e*.*g*., libraries of soluble molecules; libraries of compounds tethered to resin beads, silica chips, or other solid supports.

Nucleic acid library or array is an intentionally created collection of nucleic acids which can be prepared either synthetically or biosynthetically in a variety of different formats (e.g., libraries of soluble molecules; and libraries of oligonucleotides tethered to resin beads, silica chips, or other solid supports). Additionally, the term array is meant to include those libraries of nucleic acids which can be prepared by spotting nucleic acids of essentially any length (e.g., from 1 to about 1000 nucleotide monomers in length) onto a substrate. The term nucleic acid as used herein refers to a polymeric form of nucleotides of any length, either ribonucleotides, deoxyribonucleotides or peptide nucleic acids (PNAs), that comprise purine and pyrimidine bases, or other natural, chemically or biochemically modified, non-natural, or derivatized nucleotide bases. The backbone of the polynucleotide can comprise sugars and phosphate groups, as may typically be found in RNA or DNA, or modified or substituted sugar or phosphate groups. A polynucleotide may comprise modified nucleotides, such as methylated nucleotides and nucleotide analogs. The sequence of nucleotides may be interrupted by non-nucleotide components. Thus the terms nucleoside, nucleotide, deoxynucleoside and deoxynucleotide generally include analogs such as those described herein. These analogs are those molecules having some structural features in common with a naturally occurring nucleoside or nucleotide such that when incorporated into a nucleic acid or oligonucleotide sequence, they allow hybridization with a naturally occurring nucleic acid sequence in solution. Typically, these analogs are derived from naturally occurring nucleosides and nucleotides by replacing and/or modifying the base, the ribose or the phosphodiester moiety. The changes can be tailor made to stabilize or destabilize hybrid formation or enhance the specificity of hybridization with a complementary nucleic acid sequence as desired.

Arrays may generally be produced using a variety of techniques, such as mechanical synthesis methods or light directed synthesis methods that incorporate a combination of photolithographic methods and solid phase synthesis methods. Techniques for the synthesis of these arrays using mechanical synthesis methods are described in, e.g., U.S. Pat. Nos. 5,384,261, and 6,040,193, which are incorporated herein by reference in their entirety for all purposes. Although a planar array surface is preferred, the array may be fabricated on a surface of virtually any shape or even a multiplicity of surfaces. Arrays may be nucleic acids on beads, gels, polymeric surfaces, fibers such as fiber optics, glass or any other appropriate substrate. (See U.S. Pat. Nos. 5,770,358, 5,789,162, 5,708,153, 6,040,193 and 5,800,992, which are hereby incorporated by reference in their entirety for all purposes.)

Arrays may be packaged in such a manner as to allow for diagnostic use or can be an all-inclusive device; e.g., U.S. Pat. Nos. 5,856,174 and 5,922,591 incorporated in their entirety by reference for all purposes. Preferred arrays are commercially available from Affymetrix under the brand name GeneChip® and are directed to a variety of purposes, including genotyping and gene expression monitoring for a variety of eukaryotic and prokaryotic species. (See Affymetrix Inc., Santa Clara and their website at affymetrix.com.)

Solid support, support, and substrate are used interchangeably and refer to a material or group of materials having a rigid or semi-rigid surface or surfaces. In many embodiments, at least one surface of the solid support will be substantially flat, although in some embodiments it may be desirable to physically separate synthesis regions for different compounds with, for example, wells, raised regions, pins, etched trenches, or the like. According to other embodiments, the solid support(s) will take the form of beads, resins, gels, microspheres, or other geometric configurations. Resins may include, for example, sepharose and agarose, which may be coupled to proteins such as antibodies.

Combinatorial Synthesis Strategy: A combinatorial synthesis strategy is an ordered strategy for parallel synthesis of diverse polymer sequences by sequential addition of reagents which may be represented by a reactant matrix and a switch matrix, the product of which is a product matrix. A reactant matrix is a 1 column by m row matrix of the building blocks to be added. The switch matrix is all or a subset of the binary numbers, preferably ordered, between 1 and m arranged in columns. A binary strategy is one in which at least two successive steps illuminate a portion, often half, of a region of interest on the substrate. In a binary synthesis strategy, all possible compounds which can be formed from an ordered set of reactants are formed. In most embodiments, binary synthesis refers to a synthesis strategy which also factors a previous addition step. For example, a strategy in which a switch matrix for a masking strategy halves regions that were previously illuminated, illuminating about half of the previously illuminated region and protecting the remaining half (while also protecting about half of previously protected regions and illuminating about half of previously protected regions). It will be recognized that binary rounds may be interspersed with non-binary rounds and that only a portion of a substrate may be subjected to a binary scheme. A combinatorial masking strategy is a synthesis which uses light or other spatially selective deprotecting or activating agents to remove protecting groups from materials for addition of other materials such as amino acids.

Complementary or substantially complementary: Refers to the hybridization or base pairing between nucleotides or nucleic acids, such as, for instance, between the two strands of a double stranded DNA molecule or between an oligonucleotide primer and a primer binding site on a single stranded nucleic acid to be sequenced or amplified. Complementary nucleotides are, generally, A and T (or A and U), or C and G. Two single stranded RNA or DNA molecules are said to be substantially complementary when the nucleotides of one strand, optimally aligned and compared and with appropriate nucleotide insertions or deletions, pair with at least about 80% of the nucleotides of the other strand, usually at least about 90% to 95%, and more preferably from about 98 to 100%. Alternatively, substantial complementarity exists when an RNA or DNA strand will hybridize under selective hybridization conditions to its complement. Typically, selective hybridization will occur when there is at least about 65% complementary over a stretch of at least 14 to 25 nucleotides, preferably at least about 75%, more preferably at least about 90% complementary. See, M. Kanehisa Nucleic Acids Res. 12:203 (1984), incorporated herein by reference.

The term hybridization refers to the process in which two single-stranded polynucleotides bind non-covalently to form a stable double-stranded polynucleotide. The term hybridization may also refer to triple-stranded hybridization. The resulting (usually) double-stranded polynucleotide is a hybrid. The proportion of the population of polynucleotides that forms stable hybrids is referred to herein as the degree of hybridization.

Hybridization conditions will typically include salt concentrations of less than about 1M, more usually less than about 500 mM and less than about 200 mM. Hybridization temperatures can be as low as 5 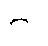 C, but are typically greater than 22 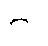 C, more typically greater than about 30 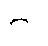 C, and preferably in excess of about 37 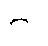 C. Hybridizations are usually performed under stringent conditions, i.e. conditions under which a probe will hybridize to its target subsequence. Stringent conditions are sequence-dependent and are different in different circumstances. Longer fragments may require higher hybridization temperatures for specific hybridization. As other factors may affect the stringency of hybridization, including base composition and length of the complementary strands, presence of organic solvents and extent of base mismatching, the combination of parameters is more important than the absolute measure of any one alone. Generally, stringent conditions are selected to be about 5 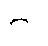 C lower than the thermal melting point (Tm) for the specific sequence at a defined ionic strength and pH. The Tm is the temperature (under defined ionic strength, pH and nucleic acid composition) at which 50% of the probes complementary to the target sequence hybridize to the target sequence at equilibrium. Typically, stringent conditions include salt concentration of at least 0.01 M to no more than 1 M Na ion concentration (or other salts) at a pH 7.0 to 8.3 and a temperature of at least 25 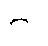 C. For example, conditions of 5X SSPE (750 mM NaCl, 50 mM NaPhosphate, 5 mM EDTA, pH 7.4) and a temperature of 25-30°C are suitable for allele-specific probe hybridizations. For stringent conditions, see for example, Sambrook, Fritsche and Maniatis. "Molecular Cloning A laboratory Manual" 2^{nd} Ed. Cold Spring Harbor Press (1989) and Anderson "Nucleic Acid Hybridization" 1^{st} Ed., BIOS Scientific Publishers Limited (1999), which are hereby incorporated by reference in its entirety for all purposes above.

Hybridization probes are nucleic acids (such as oligonucleotides) capable of binding in a base-specific manner to a complementary strand of nucleic acid. Such probes include peptide nucleic acids, as described in Nielsen et al., *Science* 254:1497-1500 (1991), Nielsen *Curr. Opin. Biotechnol.,* 10:71-75 (1999) and other nucleic acid analogs and nucleic acid mimetics. See US Patent No. 6,156,501 filed 4/3/96.

Hybridizing specifically to: refers to the binding, duplexing, or hybridizing of a molecule substantially to or only to a particular nucleotide sequence or sequences under stringent conditions when that sequence is present in a complex mixture (*e.g*., total cellular) DNA or RNA.

Probe: A probe is a molecule that can be recognized by a particular target. In some embodiments, a probe can be surface immobilized. Examples of probes that can be investigated by this invention include, but are not restricted to, agonists and antagonists for cell membrane receptors, toxins and venoms, viral epitopes, hormones (e.g., opioid peptides, steroids, etc.), hormone receptors, peptides, enzymes, enzyme substrates, cofactors, drugs, lectins, sugars, oligonucleotides, nucleic acids, oligosaccharides, proteins, and monoclonal antibodies.

Target: The term target sequence, target nucleic acid or target refers to a nucleic acid of interest. The target sequence may or may not be of biological significance. Typically, though not always, it is the significance of the target sequence which is being studied in a particular experiment. As non-limiting examples, target sequences may include regions of genomic DNA which are believed to contain one or more polymorphic sites, DNA encoding or believed to encode genes or portions of genes of known or unknown function, DNA encoding or believed to encode proteins or portions of proteins of known or unknown function, DNA encoding or believed to encode regulatory regions such as promoter sequences, splicing signals, polyadenylation signals, etc. In many embodiments a collection of target sequences is identified and assayed.

A sequence may be selected to be a target sequence if it has a region of interest and shares a characteristic with at least one other target sequence that will allow the two or more target sequences to be enriched in a subset of fragments. The region of interest may be, for example, a single nucleotide polymorphism and the shared characteristic may be, for example, that the target sequence is found on a fragment in a selected size range when a genomic sample is fragmented by digestion with a particular enzyme or enzyme combination. Collections of target sequences that each have a region of interest and share a common characteristic are particularly useful. For example, a collection of target sequences that each contain a location that is known to be polymorphic in a population and are each found on a fragment that is between 400 and 800 base pairs when human genomic DNA is digested with *Xba*I may be interrogated in a single assay. A collection may comprise from 2, 100, 1,000, 5,000, 10,000, or 50,000 to 1,000, 5,000, 10,000, 20,000, 50,000, 100,000, 1,000,000 or 3,500,000 different target sequences.

The term subset of fragments or representative subset refers to a fraction of a genome. The subset may be less than or about 0.01, 0.1, 1, 3, 5, 10, 25, 50 or 75% of the genome. The partitioning of fragments into subsets may be done according to a variety of physical characteristics of individual fragments. For example, fragments may be divided into subsets according to size, according to the particular combination of restriction sites at the ends of the fragment, or based on the presence or absence of one or more particular sequences.

Target sequences may be interrogated by any method known in the art, for example, by hybridization to an array. In some embodiments the array may be specially designed to interrogate one or more selected target sequence. The array may contain a collection of probes that are designed to hybridize to a region of the target sequence or its complement. Different probe sequences are located at spatially addressable locations on the array. For genotyping a single polymorphic site probes that match the sequence of each allele may be included. At least one perfect match probe, which is exactly complementary to the polymorphic base and to a region surrounding the polymorphic base, may be included for each allele. Multiple perfect match probes may be included as well as mismatch probes.

mRNA or mRNA transcripts: as used herein, include, but not limited to pre-mRNA transcript(s), transcript processing intermediates, mature mRNA(s) ready for translation and transcripts of the gene or genes, or nucleic acids derived from the mRNA transcript(s). Transcript processing may include splicing, editing and degradation. As used herein, a nucleic acid derived from an mRNA transcript refers to a nucleic acid for whose synthesis the mRNA transcript or a subsequence thereof has ultimately served as a template. Thus, a cDNA reverse transcribed from an mRNA, a cRNA transcribed from that cDNA, a DNA amplified from the cDNA, an RNA transcribed from the amplified DNA, *etc.,* are all derived from the mRNA transcript and detection of such derived products is indicative of the presence and/or abundance of the original transcript in a sample. Thus, mRNA derived samples include, but are not limited to, mRNA transcripts of the gene or genes, cDNA reverse transcribed from the mRNA, cRNA transcribed from the cDNA, DNA amplified from the genes, RNA transcribed from amplified DNA, and the like.

A fragment, segment, or DNA segment refers to a portion of a larger DNA polynucleotide or DNA. A polynucleotide, for example, can be broken up, or fragmented into, a plurality of segments. Various methods of fragmenting nucleic acid are well known in the art. These methods may be, for example, either chemical or physical in nature. Chemical fragmentation may include partial degradation with a DNase; partial depurination with acid; the use of restriction enzymes; intron-encoded endonucleases; DNA-based cleavage methods, such as triplex and hybrid formation methods, that rely on the specific hybridization of a nucleic acid segment to localize a cleavage agent to a specific location in the nucleic acid molecule; or other enzymes or compounds which cleave DNA at known or unknown locations. Physical fragmentation methods may involve subjecting the DNA to a high shear rate. High shear rates may be produced, for example, by moving DNA through a chamber or channel with pits or spikes, or forcing the DNA sample through a restricted size flow passage, e.g., an aperture having a cross sectional dimension in the micron or submicron scale. Other physical methods include sonication and nebulization. Combinations of physical and chemical fragmentation methods may likewise be employed such as fragmentation by heat and ion-mediated hydrolysis. *See* for example, Sambrook et al., "Molecular Cloning: A Laboratory Manual," 3^{rd} Ed. Cold Spring Harbor Laboratory Press, Cold Spring Harbor, New York (2001) (Sambrook et al.) which is incorporated herein by reference for all purposes. These methods can be optimized to digest a nucleic acid into fragments of a selected size range. Useful size ranges may be from 100, 200, 400, 700 or 1000 to 500, 800, 1500, 2000, 4000 or 10,000 base pairs. However, larger size ranges such as 4000, 10,000 or 20,000 to 10,000, 20,000 or 500,000 base pairs may also be useful.

A number of methods disclosed herein require the use of restriction enzymes to fragment the nucleic acid sample. In general, a restriction enzyme recognizes a specific nucleotide sequence of four to eight nucleotides and cuts the DNA at a site within or a specific distance from the recognition sequence. For example, the restriction enzyme *Eco*RI recognizes the sequence GAATTC and will cut a DNA molecule between the G and the first A. The length of the recognition sequence is roughly proportional to the frequency of occurrence of the site in the genome. A simplistic theoretical estimate is that a six base pair recognition sequence will occur once in every 4096 (4⁶) base pairs while a four base pair recognition sequence will occur once every 256 (4⁴) base pairs. *In silico* digestions of sequences from the Human Genome Project show that the actual occurrences may be even more infrequent for some enzymes and more frequent for others, for example, *PstI* cuts the human genome more often than would be predicted by this simplistic theory while *Sal*I and *Xho*I cut the human genome less frequently than predicted. Because the restriction sites are rare, the appearance of shorter restriction fragments, for example those less than 1000 base pairs, is much less frequent than the appearance of longer fragments. Many different restriction enzymes are known and appropriate restriction enzymes can be selected for a desired result. (For a description of many restriction enzymes *see,* New England BioLabs Catalog (Beverly, MA) which is herein incorporated by reference in its entirety for all purposes).

Information about the sequence of a region may be combined with information about the sequence specificity of a particular restriction enzyme to predict the size, distribution and sequence of fragments that will result when a particular region of a genome is digested with that enzyme. *In silico* digestion is a computer aided simulation of enzymatic digests accomplished by searching a sequence for restriction sites. *In silico* digestion provides for the use of a computer or computer system to model enzymatic reactions in order to determine experimental conditions before conducting any actual experiments. An example of an experiment would be to model digestion of the human genome with specific restriction enzymes to predict the sizes and sequences of the resulting restriction fragments.

Adaptor sequences or adaptors are generally oligonucleotides of at least 5, 10, or 15 bases and preferably no more than 50 or 60 bases in length, however, they may be even longer, up to 100 or 200 bases. Adaptor sequences may be synthesized using any methods known to those of skill in the art. For the purposes of this invention they may, as options, comprise templates for PCR primers, restriction sites, tags and promoters. The adaptor may be partially, entirely or substantially double stranded. The adaptor may be phosphorylated or unphosphorylated on one or both strands. Modified nucleotides, for example, phosphorothioates, may also be incorporated into one or both strands of an adaptor.

Adaptors are particularly useful in some embodiments of the methods if they comprise a substantially double stranded region and short single stranded regions which are complementary to the single stranded region created by digestion with a restriction enzyme. For example, when DNA is digested with the restriction enzyme *Eco*RI the resulting double stranded fragments are flanked at either end by the single stranded overhang 5'-AATT-3', an adaptor that carries a single stranded overhang 5'-AATT-3' will hybridize to the fragment through complementarity between the overhanging regions. This "sticky end" hybridization of the adaptor to the fragment may facilitate ligation of the adaptor to the fragment but blunt ended ligation is also possible.

In some embodiments the same adaptor sequence is ligated to both ends of a fragment. Digestion of a nucleic acid sample with a single enzyme may generate similar or identical overhanging or sticky ends on either end of the fragment. For example if a nucleic acid sample is digested with *Eco*RI both strands of the DNA will have at their 5' ends a single stranded region, or overhang, of 5'-AATT-3'. A single adaptor sequence that has a complementary overhang of 5'-AATT-3' can be ligated to each end of the fragment.

A single adaptor can also be ligated to both ends of a fragment resulting from digestion with two different enzymes. For example, if the method of digestion generates blunt ended fragments, the same adaptor sequence can be ligated to both ends. Alternatively some pairs of enzymes leave identical overhanging sequences. For example, *Bgl*II recognizes the sequence 5'-AGATCT-3', cutting after the first A, and *Bam*HI recognizes the sequence 5'-GGATCC-3', cutting after the first G; both leave an overhang of 5'-GATC-3'. A single adaptor with an overhang of 5'-GATC-3' may be ligated to both digestion products.

When a single adaptor sequence is ligated to both ends of a fragment the ends of a single fragment may be complementary resulting in the potential formation of hairpin structures. Formation of a base pairing interaction between the 5' and 3' ends of a fragment can inhibit amplification during PCR resulting in lowered overall yield. This effect will be more pronounced with smaller fragments than with larger fragments because the probability that the ends will hybridize is higher for smaller fragments than for larger fragments.

Digestion with two or more enzymes can be used to selectively ligate separate adapters to either end of a restriction fragment. For example, if a fragment is the result of digestion with EcoRI at one end and BamHI at the other end, the overhangs will be 5'-AATT-3' and 5'-GATC-3', respectively. An adaptor with an overhang of AATT will be preferentially ligated to one end while an adaptor with an overhang of GATC will be preferentially ligated to the second end.

Methods of ligation will be known to those of skill in the art and are described, for example in Sambrook et at. and the New England BioLabs catalog both of which are incorporated herein by reference in their entireties. Methods include using T4 DNA Ligase which catalyzes the formation of a phosphodiester bond between juxtaposed 5' phosphate and 3' hydroxyl termini in duplex DNA or RNA with blunt or sticky ends; *Taq* DNA ligase which catalyzes the formation of a phosphodiester bond between juxtaposed 5' phosphate and 3' hydroxyl termini of two adjacent oligonucleotides which are hybridized to a complementary target DNA; *E.coli* DNA ligase which catalyzes the formation of a phosphodiester bond between juxtaposed 5'-phosphate and 3'-hydroxyl termini in duplex DNA containing cohesive ends; and T4 RNA ligase which catalyzes ligation of a 5' phosphoryl-terminated nucleic acid donor to a 3' hydroxyl-terminated nucleic acid acceptor through the formation of a 3' to 5' phosphodiester bond, substrates include single-stranded RNA and DNA as well as dinucleoside pyrophosphates; or any other substrates described in the art.

A genome is all the genetic material of an organism. In some instances, the term genome may refer to the chromosomal DNA. Genome may be multichromosomal such that the DNA is cellularly distributed among a plurality of individual chromosomes. For example, in human there are 22 pairs of chromosomes plus a gender associated XX or XY pair. DNA derived from the genetic material in the chromosomes of a particular organism is genomic DNA. The term genome may also refer to genetic materials from organisms that do not have chromosomal structure. In addition, the term genome may refer to mitochondria DNA. A genomic library is a collection of DNA fragments represents the whole or a portion of a genome. Frequently, a genomic library is a collection of clones made from a set of randomly generated, sometimes overlapping DNA fragments representing the entire genome or a portion of the genome of an organism.

Chromosome refers to the heredity-bearing gene carrier of a living cell which is derived from chromatin and which comprises DNA and protein components (especially histones). The conventional internationally recognized individual human genome chromosome numbering system is employed herein. The size of an individual chromosome can vary from one type to another with a given multichromosomal genome and from one genome to another. In the case of the human genome, the entire DNA mass of a given chromosome is usually greater than about 100,000,000 bp. For example, the size of the entire human genome is about 3x10⁹ bp. The largest chromosome, chromosome no. 1, contains about 2.4x10⁸ bp while the smallest chromosome, chromosome no. 22, contains about 5.3x10⁷ bp.

A chromosomal region is a portion of a chromosome. The actual physical size or extent of any individual chromosomal region can vary greatly. The term region is not necessarily definitive of a particular one or more genes because a region need not take into specific account the particular coding segments (exons) of an individual gene.

An allele refers to one specific form of a genetic sequence (such as a gene) within a cell or within a population, the specific form differing from other forms of the same gene in the sequence of at least one, and frequently more than one, variant sites within the sequence of the gene. The sequences at these variant sites that differ between different alleles are termed variances, polymorphisms, or mutations.

At each autosomal specific chromosomal location or locus an individual possesses two alleles, one inherited from the father and one from the mother. An individual is heterozygous at a locus if it has two different alleles at that locus. An individual is homozygous at a locus if it has two identical alleles at that locus.

Polymorphism refers to the occurrence of two or more genetically determined alternative sequences or alleles in a population. A polymorphic marker or site is the locus at which divergence occurs. Preferred markers have at least two alleles, each occurring at frequency of greater than 1%, and more preferably greater than 10% or 20% of a selected population. A polymorphism may comprise one or more base changes, an insertion, a repeat, or a deletion. A polymorphic locus may be as small as one base pair. Polymorphic markers include restriction fragment length polymorphisms, variable number of tandem repeats (VNTR's), hypervariable regions, minisatellites, dinucleotide repeats, trinucleotide repeats, tetranucleotide repeats, simple sequence repeats, and insertion elements such as Alu. The first identified allelic form is arbitrarily designated as the reference form and other allelic forms are designated as alternative or variant alleles. The allelic form occurring most frequently in a selected population is sometimes referred to as the wildtype form. Diploid organisms may be homozygous or heterozygous for allelic forms. A diallelic polymorphism has two forms. A triallelic polymorphism has three forms. Single nucleotide polymorphisms (SNPs) are included in polymorphisms.

Single nucleotide polymorphism (SNPs) are positions at which two alternative bases occur at appreciable frequency (>1%) in the human population, and are the most common type of human genetic variation. The site is often preceded by and followed by highly conserved sequences of the allele (e.g., sequences that vary in less than 1/100 or 1/1000 members of the populations). A single nucleotide polymorphism may arises due to substitution of one nucleotide for another at the polymorphic site. A transition is the replacement of one purine by another purine or one pyrimidine by another pyrimidine. A transversion is the replacement of a purine by a pyrimidine or vice versa. Single nucleotide polymorphisms can also arise from a deletion of a nucleotide or an insertion of a nucleotide relative to a reference allele.

Genotyping refers to the determination of the genetic information an individual carries at one or more positions in the genome. For example, genotyping may comprise the determination of which allele or alleles an individual carries for a single SNP or the determination of which allele or alleles an individual carries for a plurality of SNPs. For example, a particular nucleotide in a genome may be an A in some individuals and a C in other individuals. Those individuals who have an A at the position have the A allele and those who have a C have the C allele. In a diploid organism the individual will have two copies of the sequence containing the polymorphic position so the individual may have an A allele and a C allele or alternatively two copies of the A allele or two copies of the C allele. Those individuals who have two copies of the C allele are homozygous for the C allele, those individuals who have two copies of the A allele are homozygous for the C allele, and those individuals who have one copy of each allele are heterozygous. The array may be designed to distinguish between each of these three possible outcomes. A polymorphic location may have two or more possible alleles and the array may be designed to distinguish between all possible combinations.

Normal cells that are heterozygous at one or more loci may give rise to tumor cells that are homozygous at those loci. This loss of heterozygosity may result from structural deletion of normal genes or loss of the chromosome carrying the normal gene, mitotic recombination between normal and mutant genes, followed by formation of daughter cells homozygous for deleted or inactivated (mutant) genes; or loss of the chromosome with the normal gene and duplication of the chromosome with the deleted or inactivated (mutant) gene.

Linkage disequilibrium or allelic association refers to the preferential association of a particular allele or genetic marker with a specific allele, or genetic marker at a nearby chromosomal location more frequently than expected by chance for any particular allele frequency in the population. For example, if locus X has alleles a and b, which occur at equal frequency, and linked locus Y has alleles c and d, which occur at equal frequency, one would expect the combination ac to occur at a frequency of 0.25. If ac occurs more frequently, then alleles a and c are in linkage disequilibrium. Linkage disequilibrium may result, for example, because the regions are physically close, from natural selection of certain combination of alleles or because an allele has been introduced into a population too recently to have reached equilibrium with linked alleles. A marker in linkage disequilibrium can be particularly useful in detecting susceptibility to disease (or other phenotype) notwithstanding that the marker does not cause the disease. For example, a marker (X) that is not itself a causative element of a disease, but which is in linkage disequilibrium with a gene (including regulatory sequences) (Y) that is a causative element of a phenotype, can be detected to indicate susceptibility to the disease in circumstances in which the gene Y may not have been identified or may not be readily detectable.

### A. Methods of Complexity reduction

Methods are provided for complexity management of nucleic acid samples, such as genomic DNA. Complexity of nucleic acids may be reduced by, for example, chemical or physical methods. The population of nucleic acids to be analyzed can be from a genomic DNA from a whole genome, a collection of chromosomes, a single chromosome or more regions from one or more chromosomes, or cloned DNA, RNA or cDNA. The genomic DNA sample of the current invention may be isolated according to methods known in the art, such as PCR, reverse transcription, and the like. It may be obtained from any biological or environmental source, including plant, animal (including human), bacteria, fungi or algae. Any suitable biological sample can be used for assay of genomic DNA. Convenient suitable samples include whole blood, tissue, semen, saliva, tears, urine, fecal material, sweat, buccal, skin and hair. The nucleic acids may be obtained from the same individual or from different individuals. When interrogating genomes it is often useful to first reduce the complexity of the sample and analyze one or more subsets of the genome. Subsets can be defined by the characteristics of the fragments such as size and nucleotide composition.

### Preferential Amplification of a Subset of Fragments Containing Target Sequences

Methods are provided for novel methods of analysis of a nucleic acid sample, such as genomic DNA. The methods include: identification and selection of a collection of target sequences; amplification of a selected subset of fragments that comprises a collection of target sequences; and, analysis of a collection of target sequences. In many embodiments a subset of fragments may be amplified by PCR wherein the subset of fragments that is amplified efficiently is dependent on the size of the fragments. In one embodiment fragmentation conditions and target sequences are selected so that the target sequences are present in the subset of fragments that are efficiently amplified by PCR. Those fragments that are efficiently amplified are enriched in the amplified sample and are present in amounts sufficient for hybridization analysis and detection using the methods disclosed. Many fragments will not be amplified enough for efficient detection using the methods disclosed and these fragments are not enriched in the amplified sample.

In many embodiments the methods include the steps of: identifying a collection of target sequences that carry sequences of interest on fragments of a selected size range; fragmenting a nucleic acid sample by digestion with one or more restriction enzymes so that the target sequences are present on fragments that are within the selected size range; ligating one or more adaptors to the fragments; and amplifying the fragments so that a subset of the fragments, including fragments of the selected size range, are enriched in the amplified product. In some embodiments the amplified sample is exposed to an array which may be specifically designed and manufactured to interrogate one or more target sequences in a collection of target sequences.

In some embodiments the size range is selected to be within the size range of fragments that can be efficiently amplified under a given set of amplification conditions. In many embodiments amplification is by PCR and the PCR conditions are standard PCR amplification conditions (*see,* for example, *PCR primer A laboratory Manual*, Cold Spring Harbor Lab Press, (1995) eds. C. Dieffenbach and G. Dveksler), under these conditions fragments that are of a predicted size range, generally less than 2 kb, will be amplified most efficiently.

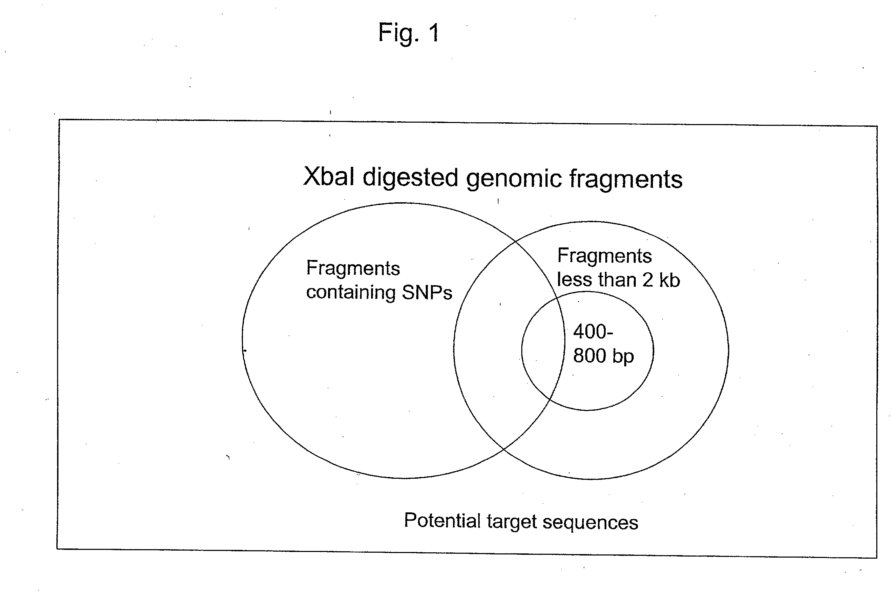

Figure 1 illustrates an example of how possible target sequences may be defined. The starting set is all of the fragments of the genome following fragmentation. Within this set there is a subset of fragments that are about 2 kb and less and would be efficiently amplified by PCR under standard conditions. Also within the starting set is a subset of fragments that contain sequences of interest, for example, fragments that contain SNPs. There is an intersection between these two subsets that represents fragments that will be efficiently amplified under standard PCR conditions and contain sequences of interest. In one embodiment these fragments are possible target sequences. In some embodiments a smaller subset is selected from within the subset of fragments that are about 2 kb and less. This subset may be, for example, fragments from about 1, 100, 200, or 400 bp to 600, 800, 1,200, 1,500 or 2,000 bp. The intersection of this subset with the subset of fragments that comprise a sequence of interest contains fragments that are potential target sequences. The set of potential target sequences will vary depending on the fragmentation method used and the size range that is selected. The collection of target sequences may comprise all potential target sequences or a further subset of the possible target sequences. Potential target sequences may be selected for the collection of target sequences or removed from the collection of target sequences based on secondary considerations such as performance in hybridization experiments, location in the genome, proximity to another target sequence in the collection, association with phenotype or disease or any other criteria that is known in the art. Additional selection criteria that may be used to select target sequences for a collection of target sequences also include, for example, clustering characteristics, whether or not a SNP is consistently present in a population, Mendelian inheritance characteristics, Hardy-Weinberg probability, and chromosomal map distribution. In one embodiment fragments that contain repetitive sequences, telomeric regions, centromeric regions and heterochromatin domains may be excluded. In one embodiment the target sequences comprise SNPs and the SNPs are selected to provide an optimal representation of the genome. For example SNPs may be selected so that the distance between SNPs in the target collection is on average between 10, 50, 100, 200 or 300 and 50, 100, 200, 400, 600 or 800 kb. Inter-SNP distances may vary from chromosome to chromosome. In one embodiment more than 80% of the SNPs in the collection of target sequence are less than about 200 kb from another SNP in the collection of target sequences. In another embodiment more than 80% of the SNPs are less than about 10, 50, 100, 150, 300 or 500 kb from another SNP in the collection of target sequences. In one embodiment SNPs that give errors across multiple families are not selected for the collection of SNPs or are removed from the analysis. In another embodiment SNPs that give ambiguous results in multiple experiments are not selected for the collection of SNPs or are removed from the analysis.

In many embodiments the methods employ the use of a computer system to assist in the identification of potential target sequences or in the selection of target sequences for a collection. For many organisms, including yeast, mouse, human and a number of microbial species, a complete or complete draft of the genomic sequence is known and publicly available. Knowledge of the sequences present in a nucleic acid sample, such as a genome, allow prediction of the sizes and sequence content of fragments that will result when the genome is fragmented under selected conditions. The pool of predicted fragments may be analyzed to identify which fragments are within a selected size range, which fragments carry a sequence of interest and which fragments have both characteristics. In some embodiments an array may then be designed to interrogate at least some of those potential target sequences. A nucleic acid sample may then be digested with the selected enzyme or enzyme and amplified under the selected amplification conditions, resulting in the amplification of the collection of target sequences. The amplified target sequences may then be analyzed by hybridization to the array. In some embodiments the amplified sequences may be further analyzed using any known method including sequencing, HPLC, hybridization analysis, cloning, labeling, etc.

In many embodiments *in silico* digestion techniques are used to identify one or more SNPs that will be present on fragments of a selected size when a genome is digested with a particular enzyme or enzyme combination. A computer may be used to locate a SNP from a public database, for example the database provided by The SNP Consortium (TSC), or within the sequence of the human genome, for example in the publicly available database such as Genbank. A computer may then be used to predict the, for example, *Bgl*II restriction sites upstream and downstream of a given SNP.

The SNPs and corresponding fragment sizes may be further separated into subsets according to fragment size. In some embodiment this step is performed by a computer or computer system. In this way a computer could be used to identify all of the SNPs that are predicted to be found on fragments that are between, for example, 200, 400, 600 or 800 and 800, 1000, 1500 or 2000 base pairs in length when a sample DNA is digested with a selected enzyme or enzyme combination.

In another embodiment the SNPs present on fragments of a selected size range following fragmentation are selected as target sequences and an array is designed to interrogate at least some of the SNPs. For example, an array may be designed to genotype some of the SNPs that are present on fragments of 400 to 800 base pairs when human genomic DNA is digested with *Xba*I. If, for example, there are 15,000 SNPs that meet these criteria a subset of these SNP, for example, 10,000 may be selected for the array. The SNPs may also be selected based on presence in a population of interest. Other empirical data such as probe performance and accuracy of allelic discrimination may be used to select SNPs for an array.

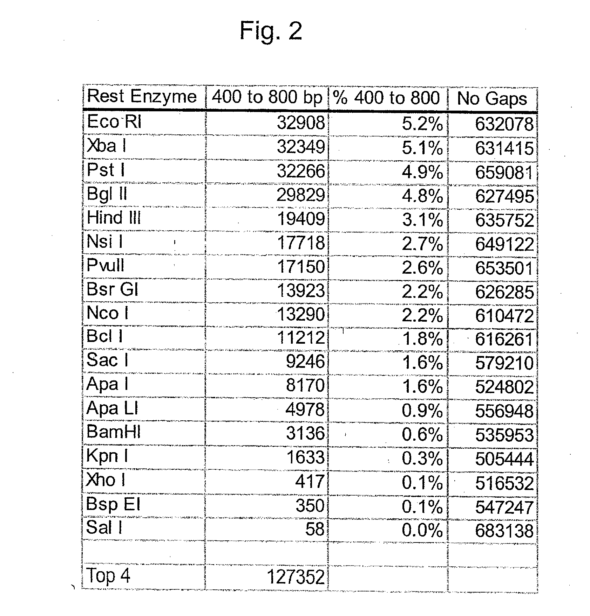

In Figure 2, *in silico* digestion was used to predict restriction fragment lengths for the more than 800,000 SNPs in the TSC database and to identify those SNP containing fragments between 400 and 800 base pairs. For example, when human genomic DNA is digested with *Eco*RI, 32,908 SNPs from the TSC database are predicted to be found on fragments between 400 and 800 base pairs. More than 120,000 of the TSC SNPs are found on fragments between 400 and 800 base pairs when genomic DNA is digested with *Eco*RI, *Xba*I, *Pst*I and *Bgl*II.

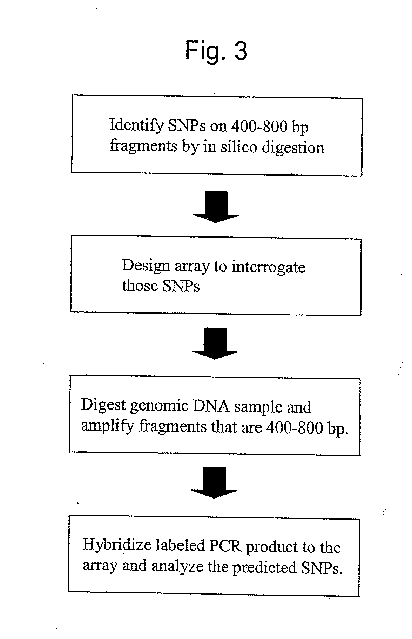

In one embodiment *in silico* prediction of the size of SNP containing fragments is combined with selection of a collection of target sequences to design genotyping assays and arrays for genotyping, see Figure 3. In one embodiment target sequences are selected from fragments that are those in the size range of 400 to 800 base pairs, but other size ranges could also be used, for example, 100, 200, 500, 700, or 1,500 to 500, 700, 1,000, or 2,000 base pairs may also be useful size ranges.

As shown in Figure 3, in this embodiment an array is designed to interrogate the SNPs that are predicted to be found in a size fraction resulting from digestion of the first nucleic acid sample with one or more particular restriction enzymes. For example, a computer may be used to search the sequence of a genome to identify all recognition sites for the restriction enzyme, *Eco*RI. The computer can then be used to predict the size of all restriction fragments resulting from an *Eco*RI digestion and to identify those fragments that contain a known or suspected SNP or polymorphism. The computer may then be used to identify the group of SNPs that are predicted to be found on fragments of, for example, 400-800 base pairs, when genomic DNA is digested with *Eco*RI. An array may then be designed to interrogate that subset of SNPs that are found on *Eco*RI fragments of 400-800 base pairs.

Arrays will preferably be designed to interrogate from 100, 500, 1000, 5000, 8000, 10,000, or 50,000 to 5,000, 10,000, 15,000, 30,000,100,000, 500,000 or 1,500,000 different SNPs. For example, an array may be designed to interrogate a collection of target sequences comprising a collection of SNPs predicted to be present on 400-800 base pair *EcoRI* fragments, a collection of SNPs predicted to be present on 400-800 base pair *Bgl*II fragments, a collection of SNPs predicted to be present on 400-800 base pair *XbaI* fragments, and a collection of SNPs predicted to be present on 400-800 base pair *Hind*III fragments. One or more amplified subsets of fragments may be pooled prior to hybridization to increase the complexity of the sample.

In some embodiments a single size selected amplification product is suitable for hybridization to many different arrays. For example, a single method of fragmentation and amplification that is suitable for hybridization to an array designed to interrogate SNPs contained on 400-800 base pair *Eco*RI would also be suitable for hybridization to an array designed to interrogate SNPs contained on 400-800 base pair *Bam*HI fragments. This would introduce consistency and reproducibility to sample preparation methods.

In some embodiments SNPs present in a collection of target sequences are further characterized and an array is designed to interrogate a subset of these SNPs. SNPs may be selected for inclusion on an array based on a variety of characteristics, such as, for example, allelic frequency in a population, distribution in a genome, hybridization performance, genotyping performance, number of probes necessary for accurate genotyping, available linkage information, available mapping information, phenotypic characteristics or any other information about a SNP that makes it a better or worse candidate for analysis.

In many embodiments a selected collection of target sequences may be amplified reproducibly from different samples or from the same sample in different reactions. In one embodiment a plurality of samples are amplified in different reactions under similar conditions and each amplification reaction results in amplification of a similar collection of target sequences. Genomic samples from different individuals may be fragmented and amplified using a selected set of conditions and similar target sequences will be amplified from both samples. For example, if genomic DNA is isolated from 2 or more individuals, each sample is fragmented under similar conditions, amplified under similar conditions and hybridized to arrays designed to interrogate the same collection of target sequences, more than 50%, more than 75% or more than 90% of the same target sequences are detected in the samples.

A given target sequence may be present in different allelic forms in a cell, a sample, an individual and in a population. In some embodiments the methods identify which alleles are present in a sample. In some embodiments the methods determine heterozygosity or homozygosity at one or more loci. In some embodiments, where SNPs are being interrogated for genotype, a genotype is determined for more than 75%, 85% or 90% of the SNPs interrogated by the array. In some embodiments the hybridization pattern on the array is analyzed to determine a genotype. In some embodiments analysis of the hybridization is done with a computer system and the computer system provides a determination of which alleles are present.

In one embodiment target sequences are selected from the subset of fragments that are less than 1,000 base pairs. An *in silico* digestion of the human genome may be used to identify fragments that are less than 1,000 base pairs when the genome is digested with the restriction enzyme, *Xba*I. The predicted *Xba*I fragments that are under 1,000 base pairs may be analyzed to identify SNPs that are present on the fragments. An array may be designed to interrogate the SNPs present on the fragments and the probes may be designed to determine which alleles of the SNP are present. A genomic sample may be isolated from an individual, digested with *Xba*I, adaptors are ligated to the fragments and the fragments are amplified. The amplified sample may be hybridized to the specially designed array and the hybridization pattern may be analyzed to determine which alleles of the SNPs are present in the sample from this individual.

In some embodiments the size range of fragments remains approximately constant, and the target sequences present in the size range vary with the method of fragmentation used. For example, if the target sequences are SNP containing fragments that are 400-800 base pairs, the fragments that meet these criteria when the human genome is digested with *Xba*I will be different than when the genome is digested with *Eco*RI, although there may be some overlap. By using a different fragmentation method but keeping the amplification conditions constant different collections of target sequences may be analyzed. In some embodiments an array may be designed to interrogate target sequences resulting from just one fragmentation condition and in other embodiments the array may be designed to interrogate fragments resulting from more than one fragmentation condition. For example, an array may be designed to interrogate the SNPs present on fragments that are less than 1,000 base pairs when a genome is digested with *XbaI* and the SNPs present on fragments that are less than 1,000 base pairs when a genome is digested with *Eco*RI.

In many embodiments an enzyme is selected so that digestion of the sample with the selected enzyme followed by amplification results in a sample of a complexity that may be specifically hybridized to an array under selected conditions. For example, digestion of the human genome with *Xba*I, *Eco*RI or *Bgl*II and amplification with PCR reduces complexity of the sample to approximately 2%. In another embodiment the sample is digested with an enzyme that cuts the genome at frequencies similar to *Xba*I, *Eco*RI or *Bgl*II, for example, *Sac*I, *Bsr*GI or *Bcl*I. Different complexity levels may be used. Useful complexities range from 0.1, 2, 5, 10 or 25% to 1, 2, 10, 25 or 50% of the complexity of the starting sample. In some embodiments the complexity of the sample is matched to the content on an array.

In many embodiments the target sequences are a subset that is representative of a larger set. For example, the target sequences may be 1,000, 5,000, 10,000 or 100,000 to 10,000, 20,000, 100,000, 1,500,000 or 3,000,000 SNPs that maybe representative of a larger population of SNPs present in a population of individuals. The target sequences may be dispersed throughout a genome, including for example, sequences from each chromosome, or each arm of each chromosome. Target sequences may be representative of haplotypes or particular phenotypes or collections of phenotypes. For a description of haplotypes *see,* for example, Gabriel et al., Science, 296:2225-9 (2002), Daly et al. Nat Genet., 29:229-32 (2001) and Rioux et al., Nat Genet., 29:223-8 (2001), each of which is incorporated herein by reference in its entirety.

The methods may be combined with other methods of genome analysis and complexity reduction. Other methods of complexity reduction include, for example, AFLP, *see* US Patent 6,045,994, which is incorporated herein by reference, and arbitrarily primed-PCR (AP-PCR) *see* McClelland and Welsh, in *PCR Primer: A laboratory Manual*, (1995) eds. C. Dieffenbach and G. Dveksler, Cold Spring Harbor Lab Press, for example, at p 203, which is incorporated herein by reference in its entirety. Additional methods of sample preparation and techniques for reducing the complexity of a nucleic sample are described in Dong et al., *Genome Research* 11, 1418 (2001), in U.S. Patent No 6,361,947, 6,391,592 and U.S. Patent application Nos. 09/512,300, 09/916,135, 09/920,491, 09/910,292, 10/013,598 and 10/264,945, which are incorporated herein by reference in their entireties.

One method that has been used to isolate a subset of a genome is to separate fragments according to size by electrophoresis in a gel matrix. The region of the gel containing fragments in the desired size range is then excised and the fragments are purified away from the gel matrix. The SNP consortium (TSC) adopted this approach in their efforts to discover single nucleotide polymorphisms (SNPs) in the human genome. *See,* Altshuler *et al*., *Nature* 407: 513-516 (2000) and The International SNP Map Working Group, *Nature* 409: 928-933 (2001) both of which are herein incorporated by reference in their entireties for all purposes.

PCR amplification of a subset of fragments is an alternative, non-gel-based method to reduce the complexity of a sample. PCR amplification in general is a method of reducing the complexity of a sample by preferentially amplifying one or more sequences from a complex sample. This effect is most obvious when locus specific primers are used to amplify a single sequence from a complex sample, but it is also observed when a collection of sequences is targeted for amplification.

There are many known methods of amplifying nucleic acid sequences including e.g., PCR. *See,* e.g., *PCR Technology: Principles and Applications for DNA Amplification* (ed. H.A. Erlich, Freeman Press, NY, NY, 1992); *PCR Protocols: A Guide to Methods and Applications* (eds. Innis, et al., Academic Press, San Diego, CA, 1990); Mattila et al., *Nucleic Acids Res*. 19, 4967 (1991); Eckert et al., *PCR Methods and Applications* 1, 17 (1991); *PCR* (eds. McPherson et al., IRL Press, Oxford); and U.S. Patent 4,683,202, 4,683,195, 4,800,159, 4,965,188 and 5,333,675 each of which is incorporated herein by reference in their entireties for all purposes.

PCR is an extremely powerful technique for amplifying specific polynucleotide sequences, including genomic DNA, single-stranded cDNA, and mRNA among others. Various methods of conducting PCR amplification and primer design and construction for PCR amplification will be known to those of skill in the art. Generally, in PCR a double stranded DNA to be amplified is denatured by heating the sample. New DNA synthesis is then primed by hybridizing primers to the target sequence in the presence of DNA polymerase and excess dNTPs. In subsequent cycles, the primers hybridize to the newly synthesized DNA to produce discreet products with the primer sequences at either end. The products accumulate exponentially with each successive round of amplification.

The DNA polymerase used in PCR is often a thermostable polymerase. This allows the enzyme to continue functioning after repeated cycles of heating necessary to denature the double stranded DNA. Polymerases that are useful for PCR include, for example, *Taq* DNA polymerase, *Tth* DNA polymerase, *Tfl* DNA polymerase, *Tma* DNA polymerase, *Tli* DNA polymerase, *Pfx* DNA polymerase and *Pfu* DNA polymerase. There are many commercially available modified forms of these enzymes including: AmpliTaq®, AmpliTaq® Stoffel Fragment and AmpliTaq Gold® available from Applied Biosystems (Foster City, CA). Many are available with or without a 3- to 5' proofreading exonuclease activity. See, for example, Vent® and Vent® (exo-) available from New England Biolab (Beverly, MA).

Other suitable amplification methods include the ligase chain reaction (LCR) *(e.g.,* Wu and Wallace, *Genomics* 4, 560 (1989) and Landegren et al., *Science* 241, 1077 (1988)), transcription amplification (Kwoh et al., *Proc. Natl. Acad. Sci. USA* 86, 1173 (1989)), self-sustained sequence replication (Guatelli et al., *Proc. Nat. Acad. Sci. USA,* 87, 1874 (1990)) and nucleic acid based sequence amplification (NABSA). (*See*, US patents nos. 5,409,818, 5,554,517, and 6,063,603 each of which is incorporated herein by reference in their entireties).

When genomic DNA is digested with one or more restriction enzymes the sizes of the fragments are randomly distributed over a broad range. Following adaptor ligation, all of the fragments that have adaptors ligated to both ends will compete equally for primer binding and extension regardless of size. However, standard PCR typically results in more efficient amplification of fragments that are smaller than 2.0 kb. (*See* Saiki et al. *Science* 239, 487-491 (1988) which is hereby incorporated by reference it its entirety). The natural tendency of PCR is to amplify shorter fragments more efficiently than longer fragments. This inherent length dependence of PCR results in efficient amplification of only a subset of the starting fragments. Those fragments that are smaller than 2 kb will be more efficiently amplified than larger fragments when a standard range of conditions are used. This effect may be related to the processivity of the enzyme, which limits the yield of polymerization products over a given unit of time. The polymerase may also fail to complete extension of a given template if it falls off the template prior to completion. What is observed is that longer templates are less efficiently amplified under a standard range of PCR conditions than shorter fragments. Because of the geometric nature of PCR amplification, subtle differences in yields that occur in the initial cycles will result in significant differences in yields in later cycles. (*See*, PCR Primer: A Laboratory Manual, CSHL Press, Eds. Carl Dieffenbach and Gabriela Dveskler, (1995), (Dieffenbach et al.) which is herein incorporated by reference in its entirety for all purposes.) Variations in the reaction conditions such as, for example, primer concentration, extension time, salt concentration, buffer, temperature, and number of cycles may alter the size distribution of fragments to some extent. Inclusion of chain terminating nucleotides or nucleotide analogs may also alter the subset of fragments that are amplified. (*See*, Current Protocols in Molecular Biology, eds. Ausubel et al. (2000), which is herein incorporated by reference in its entirety for all purposes.) The presence or absence of exonuclease activity may also be used to modify the subset of fragments amplified. (*See*, for example, *PCR Strategies,* eds. Innis et al, Academic Press (1995), (Innis et al.),which is herein incorporated by reference for all purposes).

Ligation of a single adapter sequence to both ends of the fragments may also impact the efficiency of amplification of smaller fragments due to the formation of pan-handle structures between the resulting terminal repeats, *see*, for example, Qureshi et al. *GATA* 11(4): 95-101, (1994), Caetano-Anolles et al. *Mol. Gen. Genet.* 235: 157-165 (1992) and Jones and Winistorfer, *PCR Methods Appl*. 2:197-203 (1993). Smaller fragments are more likely to form the pan-handle structure and the loop may be more stable than longer loops.

In some embodiments sequences that are on smaller fragments, for example, fragments less than 400 bp or less than 200 bp are not selected as target sequences. In addition to the bias against amplification of these fragments when a single adapter is used there are also fewer small fragments following fragmentation with a restriction enzyme or enzymes. For many enzymes fragments that are, for example, smaller than 200 base pairs are relatively rare in the sample being amplified because of the infrequency of the recognition site. Since the small fragments are rare and account for relatively little sequence information there is also a decreased probability that sequences of interest will be present on small fragments.

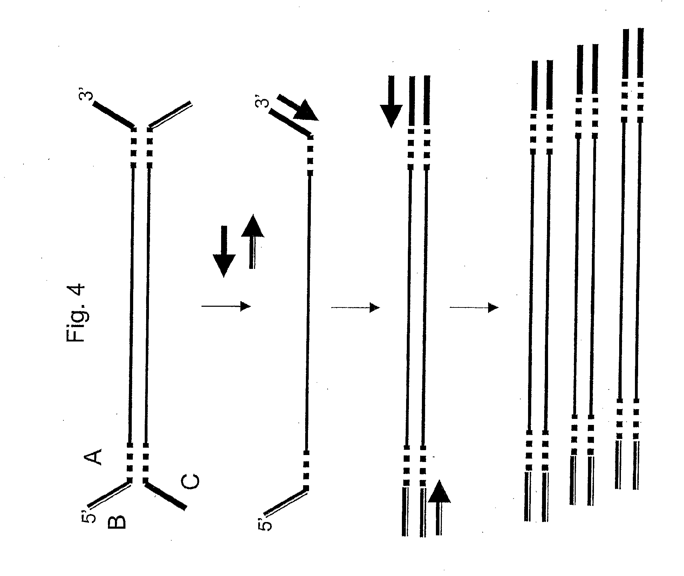

In some embodiments the potential for formation of a stable duplex between the ends of the fragment strands is reduced. In one embodiment the adapter contains internal mismatches. In another embodiment the two strands of the adapter have a region of complementarity and a region of non-complementarity (Figure 4). The region of complementarity (A and A') is near the end that will ligate to the fragments. The fragments can be amplified using primers to the non-complementary regions (B and C). Amplified products will have sequences B and C at the ends which will destabilize basepairing between A and A'. In another embodiment the sample may be amplified with a single primer for at least some of the cycles of amplification.

In some embodiments two or more different adaptors are ligated to the ends of the fragments. Ligation of different adaptor sequences to the fragments may result in some fragments that have the same adaptor ligated to both ends and some fragments that have two different adaptors ligated to each end. Small fragments that have different adaptors ligated to each end are more efficient templates for amplification than small fragments that have the same adaptor ligated to both ends because the potential for base pairing between the ends of the fragments is eliminated or reduced.

In one embodiment, the fragmented sample is fractionated prior to amplification by, for example, applying the sample to a gel exclusion column. Adaptors may be ligated to the fragments before or after fractionation. For example, to exclude the shortest fragments from the amplification the fragments can be passed over a column that selectively retains smaller fragments, for example fragments under 400 base pairs. The larger fragments may be recovered in the void volume. Because the shortest fragments in the PCR would be approximately 400 base pairs, the resulting PCR products will primarily be in a size range larger than 400 base pairs.

The materials for use in the present invention are ideally suited for the preparation of a kit suitable for obtaining an amplified collection of target sequences. Such a kit may comprise various reagents utilized in the methods, preferably in concentrated form. The reagents of this kit may comprise, but are not limited to, buffer, appropriate nucleotide triphosphates, appropriate dideoxynucleotide triphosphates, reverse transcriptases, nucleases, restriction enzymes, adaptors, ligases, DNA polymerases, primers, instructions for the use of the kit and arrays.

In order to interrogate a whole genome it is often useful to amplify and analyze one or more representative subsets of the genome. There may be more than 3,000,000 SNPs in the human genome, but tremendous amounts of information may be obtained by analysis of a subset of SNPs that is representative of the whole genome. Subsets can be defined by many characteristics of the fragments. In one embodiment, the subsets are defined by the proximity to an upstream and downstream restriction site and by the size of the fragments resulting from restriction enzyme digestion. Useful size ranges may be from 100, 200, 400, 700 or 1000 to 500, 800, 1500, 2000, 4000 or 10,000. However, larger size ranges such as 4000, 10,000 or 20,000 to 10,000, 20,000 or 500,000 base pairs may also be useful.

In one embodiment Fragment Selection by PCR (FSP) as described in U.S. Patent application Nos. 09/916,135 and 20030036069 is used to reduce complexity. In another embodiment the primer or adapter sequences used for FSP are derived from sequences on the GenFlex Tag Array (Affymetrix, Inc.), see, for example, U. S. Patent No. 6,458,530 and U.S. Patent application serial 09/827,383 which are incorporated herein by reference.

Complexity of nucleic acids may be reduced by, for example, physical methods including separation based on physical properties of the sample. Physical fragmentation methods may involve subjecting the DNA to a high shear rate. High shear rates may be produced, for example, by moving DNA through a chamber or channel with pits or spikes, or forcing the DNA sample through a restricted size flow passage, e.g., an aperture having a cross sectional dimension in the micron or submicron scale.

Any other non-destructive method of isolating DNA fragments of the desired size may be employed. For example, size-based chromatography, HPLC, dHPLC or a sucrose density gradient could be used to reduce the DNA pool to those fragments within a particular size range and then this smaller pool could be run on an electrophoresis gel.

Physical properties of the sample that may be used for separation include, for example, charge, molecular weight, hydrophobicity, and content of specific nucleotides, for example, GC content. Separation may be accomplished by electrophoresis in a gel matrix, for example agarose gel electrophoresis or polyacrylamide electrophoresis. One method that has been used to isolate a subset of a genome is to separate fragments according to size by electrophoresis in a gel matrix. The region of the gel containing fragments in the desired size range is then excised and the fragments are purified away from the gel matrix. The SNP consortium (TSC) adopted this approach in their efforts to discover single nucleotide polymorphisms (SNPs) in the human genome. See, Altshuler et al., *Nature* 407: 513-516 (2000) and The International SNP Map Working Group, *Nature* 409: 928-933 (2001) both of which are herein incorporated by reference in their entirety for all purposes.

Size exclusion columns may be used to reduce complexity. Another method of complexity reduction that may be used is separation of the sample in a matrix followed by isolation of a portion of the matrix containing a subset of the sample. The subset may then be separated substantially from the matrix.

In another embodiment, an initial population of nucleic acid is treated so as to reduce or eliminate fragments having repetitive sequences such as the *Alu I*, LINE-1 repeats of human DNA. Repeat sequences are sequences that occur more than once in haploid genome of an organism. More than 30% of human DNA consists of sequences that repeat at least 20 times. In general, nonrepeat sequences contain the coding and key regulatory regions of genomic DNA and are of interest fro more subsequent genetic analysis. Moreover, repetitive sequences, if present in the target DNA, have low complexity and high concentration and therefore hybridize faster than single copy elements resulting in non-specific hybridization. Repeat sequences can be eliminated by a process that involves preincubating target DNA with Cot-1 DNA (i.e. DNA with a Cot value of 1.0). Nucleic acids that associate with the Cot-1 DNA may be removed by chromatographic methods or any other method known in the art. The Cot-1 DNA may for example be attached to a solid support such as a bead. The nucleic acid sample is incubated with the Cot-1 DNA attached to the solid support under hybridization conditions and the DNA that hybridizes to the Cot-1 DNA is removed by removing the solid support. Cot-1 DNA has been successfully used to remove repetitive sequences from library probes used in FISH (fluorescence in situ hybridization). Before performing a genetic analysis using a nucleic acid probe array, it would be clearly advantageous if the target DNA does not comprise the entire complexity of a large genome, but instead comprises a representative sample highly enriched in single copy or coding sequences.

In one embodiment complexity is reduced by selecting transcriptionally active regions of the genome. This may be done, for example, by immunoprecipitation of active chromatin using antibodies that recognize active chromatin. Particular histone modifications such as histone acetylation are thought to be associated with specific chromatin regions in eukaryotic cells determining their transcriptional activity (K. Struhl, *Genes Dev*., vol. 12, 599 (1998); M. Grunstein, *Nature,* vol. 389, 349 (1997) both of which are incorporated herein by reference). The chromosomal immunoprecipitation (ChIP) assay has been demonstrated as a method which successfully allows for the purification of in vivo protein/protein interactions which occur in combination with DNA regulatory elements as well as direct protein/DNA interactions from cellular extracts of either cytoplasmic or nuclear origin (Solomon et al., *Cell,* 1988, 53: 937-947; de Belle et al., *Biotechniques,* 2000,29 (1): 170-175 both of which are incorporated herein by reference). Immunoprecipitating a protein/DNA complex will involve the utilization of an antibody of either polyclonal or monoclonal origin to directly and specifically recognize and bind to acetylated or non-acetylated histones for instance. This will allow the extraction of a protein/DNA complex from a bulk population of crosslinked protein/DNA complexes and the selection of transcriptionally active regions of the genome.

In another embodiment complexity is reduced by separation of chromosomes into subsets. This may be accomplished, for example, by pulse field gel electrophoresis (PFGE- See "Separation of Yeast Chromosome-sized DNAs by Pulsed Field Gradient Gel Electrophoresis," *Cell*, 37: 67-75; (1984) and in U.S. Pat. No. 4,473,452). In particular, PFGE allows the resolution of extremely large DNA, raising the upper size limit of DNA separation in agarose from 30-50 kb to well over 10 Mb (10,000 kb) by alternatively activating two differently oriented electric fields. The discontinuous electric field forces the DNA molecules to change their conformation and direction of migration during the electrophoresis and therefore allow the fractionation of large DNA fragments. PFGE permits cloning and analysis of a smaller number of very large pieces of a genome instead of cloning a large number of small fragments of DNA. (see US 5,135,628, Viskochil D et al. (1990), *Cell*, 62:187).

In another embodiment one or more chromosomes are selected by association of the chromosome to be selected with a solid support such as an array or a bead. The array or bead may have an affinity for one or more chromosomes of interest.

In another embodiment somatic cell hybrids are used to reduce complexity. The reduced complexity sample may be derived from a cell or tissue that contains genes from species other than humans in addition to the human gene that is the template for producing a probe (such as a somatic cell hybrid, Weiss M.C. and Green H. *Proc. Natl. Acad. Sci. USA* , 58: 1104-1111 (1967)). Somatic cells from two animal species such as human and rodents may be fused to form a hybrid cell in culture. Nuclei can subsequently fuse to form a somatic-cell hybrid and hybrid cells are isolated using selectable markers. The resulting hybrid cells may contain a complete set of genes from a species other than humans and one or more human chromosome segments or chromosomes. The cell may contain more than one human chromosome, for example, the cell may contain at least 5 human chromosomes, or, at least 15 or 20 human chromosomes, or, all human autosomal chromosomes present in a single parental copy. The cell may also contain one copy of the human X chromosome or one copy of the human Y chromosome. The hybrid cell line established can then be utilized to detect the presence of genes and map them on the remaining human chromosome. Moreover, somatic cell hybrids have been shown to maintain the active or inactive state of the X chromosome and may maintain the characteristics of imprinted genes such as monoallelic expression and differential methylation of maternal and paternal alleles (Gabriel J.M. et al., *Proc. Natl. Acad. Sci. USA* (1998) 95:14857). Methods of preparing hybrid cell lines which contain whole or fragments of heterologous eukaryotic chromosomes are known in the art. These techniques include, for example, microcell-mediated transfer of chromosomes into somatic cells (R.E.K. Fournier and F.H. Ruddle (1977), *Proc. Natl. Acad. Sci. USA,* 74:319), somatic cell hybridization (Ruddle and Creagan (1975), *Ann. Rev. Genet.*, 9:407), chromosome-mediated gene transfer (McBride and Ozer (1973), *Proc Natl. Acad. Sci. USA,* 70:1258), DNA-mediated gene transfer (Wigler et al., (1978), *Cell*,14:725, and irradiation-fusion techniques (Goss and Harris (1975), *Nature*, 255:680, Benham et al. (1989) *Genomics*, 4:509; Cox et al. (1990), *Science,* 250:245).

In one embodiment complexity is reduced by positive selection for a subset of a genome. In another embodiment complexity is reduced by negative selection for a subset of a genome. Other complexity reduction methods that could be used for genotyping include the use of negative selection to remove unwanted sequences from the sample. Sequences could be selected for removal based on a number of criteria, for example, fragments of a certain size could be removed or sequences that contain particular sequences could be removed. The sequences to be removed may be removed, for example, by hybridization to an array or a bead.

Other methods of complexity reduction are described in U.S. Patent Nos. 6,361,947 and 6,458,530 and U.S. Patent application Nos. 09/916,135, 09/920,491, 09/910,292, 10/013,598 and 10/264,945 and publication number 20030036069.

In another embodiment complexity is reduced by phase separation methods. For example, solvent may be added to distribute DNA or to enrich for DNA in the soluble phase.

For example, an individual genomic DNA segment from the same genomic location as a designated reference sequence can be amplified by using primers flanking the reference sequence. Multiple genomic segments corresponding to multiple reference sequences can be prepared by multiplex amplification including primer pairs flanking each reference sequence in the amplification mix. Alternatively, the entire genome can be amplified using random primers (typically hexamers) (see Barrett et al., *NAR*, 23:3488-3492 (1995)) or by fragmentation and reassembly (see, e.g., Stemmer et al., *Gene,* 164:49-53 (1995)).

Prior to or concurrent with genotyping the genomic sample may be amplified by a variety of mechanisms, some of which may employ PCR. The sample may be amplified on the array, see, for example, U.S. Patent No. 6,300,070 and U.S. patent application 09/513,300 which are incorporated herein by reference.

In some embodiments of the invention the reduced complexity sample is amplified as part of complexity reduction. In some embodiments the reduced complexity sample is amplified following complexity reduction. In some embodiments the reduced complexity sample is not amplified. As mentioned previously, the nucleic acid samples can be amplified before or after enrichment.

Another method to reduce complexity of a nucleic acid sample is to fragment the nucleic acid, for example, with restriction enzymes, ligate adaptors to the ends of the fragments, digest the fragments to produce single stranded half molecules, make the single stranded half molecules double stranded (See U.S. application 20030039069, incorporated herein by reference in its entity for all purposes).

In one embodiment many individuals are assayed in a highly multiplexed or highly parallel manner.

In another embodiment materials that may be used in one or more embodiments are combined in a kit.

### B. Genotyping Reduced Complexity Samples

Some embodiments of the present methods relate to means for the detection of signals to genotype reduced complexity samples. In addition methods of using genotyping information are disclosed, as well as uses for genotyping arrays.

In one embodiment an array of probes attached to a solid support is used. The solid support may be a disposable device including arrays, microparticles, beads and magnetic particles. In some embodiments, the methods relate to the detection and characterization of nucleic acid sequences and variations in nucleic acid sequences. Various methods are known to the art which may be used to detect and characterize specific nucleic acid sequences and sequence variants.

In currently employed hybridization assays, the target nucleic acid must be labeled with a detectable label (where the label may be either directly or indirectly detectable), such that the presence of probe/target duplexes can be detected following hybridization. Suitable labels may provide signals detectable by luminescence, radioactivity, colorimetry, x-ray diffraction or absorption, magnetism or enzymatic activity, and may include, for example, fluorophores, chromophores, radioactive isotopes, enzymes, and ligands having specific binding partners. Currently employed labels include isotopic and fluorescent labels, where fluorescent labels are gaining in popularity as the label of choice, particularly for array based hybridization assays.

The complexity reduction methods disclosed, herein, are particularly useful when combined with methods to detect the genotype of a sample. In many embodiments the reduced complexity sample is genotyped using an array of probes.

The reduced complexity sample may be genotyped directly or subjected to further sample preparation methods, such as single base extension or multiplex PCR amplification. In another embodiment the complexity reduction methods disclosed may be combined with multiplex amplification with locus specific primers.

In one embodiment subsets may be exposed to an array which may have been specifically designed and manufactured to interrogate the isolated sequences. Design of both the complexity management steps and the arrays may be aided by computer modeling techniques. Generally, the steps of the present invention involve reducing the complexity of a nucleic acid sample using the disclosed techniques alone or in combination. The allele that is present at a polymorphic location may be detected by a variety of means many of which would benefit from a complexity reduction step prior to the analysis of the sequence of the polymorphic base. Complexity reduction eliminates sequence that is not of interest, for example sequence that does not contain polymorphism and sequence that is repetitive. Removal of sequence that is not of interest results in enrichment of sequence that is of interest. For many detection methods this enrichment of sequences of interest improves the efficiency of the detection method and allows genotyping using reduced amounts of sample, improves the efficiency of genotyping and increases the accuracy of the results.

In many of the embodiments of the present invention the identity of polymorphic alleles are identified by hybridization to allele specific probes. Hybridization may be detected by any method known in the art. In one embodiment nucleotide analogues are used to enhance discrimination and reduce non-specific hybridization. In one embodiment hybridizations are done in small volumes to increase the kinetics of binding. In another embodiment an electrical current is used to reduce non-specific hybridization. In one embodiment hybridization rate enhancers are used.

In one embodiment an energy transfer based hybridization or beacon/detector technology such as that described in Gionata Leone, et al., "Molecular beacon probes combined with amplification by NASBA enable homogeneous, real-time detection of RNA", *Nucleic Acids Research*, 26 :2150-2155 (1988), which is incorporated herein by reference, may be used. Molecular beacons are probes labeled with fluorescent moieties where the fluorescent moieties fluoresce only when the detection probe is hybridized (Tyagi and Kramer, *Nature Biotechnology* 14:303-308 (1996), "Molecular beacons: probes that fluoresce upon hybridization" and Tyagi et al., "Multicolor molecular beacons for allele discrimination" *Nat Biotechnol.* 1998, 16:49 which are incorporated herein by reference). The use of such probes eliminates the need for removal of unhybridized probes prior to label detection because the unhybridized detection probes will not produce a signal. This may also be used for multiplex assays. The beacons/detectors allow homogeneous detection of a target sequence and may be composed of a stem-loop structure in which the loop portion contains the sequence complementary to the target. In the presence of target, the stem of the beacon/detector structure is "opened" and the loop hybridizes to the target. The "open" beacon/detector produces fluorescence that normally is quenched in the "closed" state. According to one embodiment of the present invention, a portion of the beacons/detectors are immobilized onto a solid support. The beacons/detectors may be constructed in such a way that the reaction conditions open the stem structure sequence. The target is brought into contact with the beacons/detectors to thereby allow the stem structure of the beacons/detectors to open and hybridization to the loop structure to occur. If target is present the open structure moves the quencher away from the detector molecule allowing the label to be detected. In one embodiment the complexity reduction methods of the present invention are used to prepare substrates for Taqman analyses.

In another embodiment the identity of an allele is detected by an electrical pore analysis. Nucleic acid may be sequenced at single-nucleotide resolution by coupling sensitive detectors to nanopores. Individual DNA molecules may be sequenced at rates of up to 1000 bases per second, eliminating the need for amplification. Bases on a single-stranded DNA molecule are forced, under an electric potential difference, single-file through a nanopore less than 2 nm in diameter. An integral detector in the pore translates the characteristic physical and chemical properties of a base or sequence of bases into an electrical signature. When a pore is occupied by polynucleotides, the ionic conductance decreases according to the nucleotide composition of the DNA. See, D. W. Deamer, M. Akeson, *Trends Biotechnol.* 18, 147 (2000). Reducing the complexity of a sample or enriching the sample for sequences of interest prior to electrical pore analysis may be used to improve efficiency and improve signal to noise ratios. In another embodiment the identity of an allele may be detected by an electrical pore analysis such as that described in Baldarelli et al. (U.S. Pat. No. 6,015,714) and in Church et al. (U.S. Pat. No. 5,795,782). These inventors describe the use of small pores (nanopores) to characterize polymers including DNA and RNA molecules on monomer by monomer basis. In particular, Baldarelli et al. characterize and sequence nucleic acid polymers by passing a nucleic acid through a channel (or pore). The channel is imbedded in an interface which separates two media. As the nucleic acid molecule passes through the channel, the nucleic acid alters an ionic current by blocking the channel. As the individual nucleotides pass through the channel, each base/nucleotide alters the ionic current in a manner which allows one to identify the nucleotide transiently blocking the channel, thereby allowing one to determine the nucleotide sequence of the nucleic acid molecule. In US Patent application No 2002013789, which is herein incorporated by reference in their entirety for all purposes, nucleic acids present in a fluid sample are translocated through a "nanopore", e.g. by application of an electric field to the fluid sample. The current amplitude through the nanopore is monitored during the translocation process and changes in the amplitude are related to the passage of single- or double-stranded molecules through the nanopore. The measured data values, e.g. current amplitudes, are then manipulated to produce a current blockade profile or similar output capable of being compared against reference outputs such that the nature of the nucleic acid, i.e. the single or double strandedness of the nucleic acid passing through the pore can be determined. In one embodiment, comparison of the observed total current blockade profiles to reference current blockade profiles of single and double stranded nucleic acids allows the determination of the presence of double stranded nucleic acids in a sample and may be use to detect hybridization events in assays where complementary nucleic acids are hybridized to each other. The presence of double-stranded nucleic acids indicates that hybridization between the probe and target has occurred.

The complexity reduction methods disclosed may also be combined with other single-molecule analysis techniques, such as, atomic force microscopy (AFM). AFM involves scanning a nanometer-scale tip across a surface (see, J. Binnig et al., *Phys. Rev. Lett.* 56, 930 (1986)). To read the surface of DNA. Intermolecular forces between the tip and surface move a flexible cantilever up and down. The corresponding deflections are measured with a laser, and a topographic map of the surface is generated. AFM may be used to scan single-stranded DNA with single-nucleotide resolution to analyze genotypes.

The complexity reduction methods disclosed may also be combined with other allele detection methods including but not limited to allele detection by raman scattering, allele detection by electrical conductance, MALDI-TOF (Matrix-assisted laser desorption ionization -time-of-flight) mass spectrometry mass spectrometry, pyrosequencing and allele detection by the use of an e-sensor.

In another embodiment the identity of an allele is detected by electrical conductance. In general, a double strand of polynucleotide is about one million times more electrically conductive than a single strand and measure of conductivity may be used as an efficient hybridization test on DNA chips (see Okahata et al.(1998), *Supramol. Sci.,* 5:317 and Kasumov et al., (2001), *Science,* 291:280, which are herein incorporated by reference in their entirety for all purposes). Moreover, since electrical current is sensitive to base pairing and measure of current flow can be used to detect mispairing.

In one embodiment the identity of an allele is detected by the use of an e-sensor. An e-sensor links biological macromolecules and electronic circuitry and proceeds via a sandwich hybridization assay. The e-sensor DNA detection system employs two single-stranded DNA probes: a capture probe that immobilizes the target to the gold electrode surface and a signaling probe. Motorola's eSensor™, for example, utilizes a ferrocene-conjugated signaling oligonucleotide. Binding the target DNA to both probes brings the ferrocene moieties in close proximity to the electrode surface and electrons flow to the electrode surface only when the target is present and specifically hybridized to both signaling probe and capture probe. The current generated is directly proportional to the number of ferrocene moieties immobilized at the electrode surface (e.g. quantity of probe that is bound). Generated current may be measured to determine which allele or alleles are present.

Matrix-assisted laser desorption ionization (MALDI) time-of-flight (TOF) mass spectrometry has been adapted primarily for detecting sequence variations between individuals. It uses the chemistry established for conventional sequencing but replaces the size separation of DNA in gels with mass-dependent strand separation of gas phase ions in a vacuum. The chemistry specifies the base at the end of a fragment, and the mass of the fragment specifies the location of the corresponding base in the sequence. This approach, permits simultaneous analysis of many DNA strands in seconds.

Pyrosequencing is a nonelectrophoretic DNA sequencing method used primarily for mutation analysis and genotyping. It enables sequencing of DNA strands up to 200 nucleotides long and is currently one of the fastest methods for analyzing a primed DNA strand. The technique takes advantage of four enzymes [DNA polymerase, sulfurylase (from yeast), luciferase (from the firefly), and apyrase (from potato)] cooperating in a single tube to signal the incorporation of a nucleotide to a growing DNA strand. Detection is based on the visible light produced by coupling the pyrophosphate released during nucleotide incorporation with the enzymes sulfurylase and luciferase. By sequentially adding nucleotides and observing the flash of light each addition causes, the sequence of the template can be determined as the DNA strand is copied (see, M. Ronaghi, *Genome Res.* 11, 3 (2001)).

In another embodiment the identity of an allele is detected by Raman scattering. Surface enhanced Raman scattering has been investigated as a method for detecting and identifying single base differences in double stranded DNA fragments (see Chumanov, G. "Surface Enhanced Raman Scattering for Discovering and Scoring Single Based Differences in DNA" Proc. Volume SPIE, 3608 (1999)). SERS principles have also been used in the development of gene probes which do not require the use of radioactive labels. These probes can be used to detect DNA via hybridization to a DNA sequence complementary to the probe. (See Vo-Dinh, T. "Surface-Enhanced Raman Gene Probes" *Anal. Chem.* 66:3379-3383 (1994)). Coupling near-field optics with SERS techniques, US Patent 6,376,177 describes an analytical method for determining whether a DNA sample comprises double-stranded DNA by analyzing the DNA sample by near field Raman spectroscopy to determine whether the sample produces low frequency vibrations. The presence of these vibrations indicates the presence of double stranded DNA in the DNA sample (e.g., hybridized DNA fragments).

Electrochemical DNA biosensors are attractive device for converting the hybridization events into an analytical signal for obtaining sequence-specific information. The electrochemical detection of nucleic acids provides an alternative to fluorescent bioassay techniques that potentially eliminates the need for labeling (Johnston, D. H., Glasgow, K. C. and Thorp, H. H., "Electrochemical Measurement of the Solvent Accessibility of Nucleobases Using Electron Transfer between DNA and Metal Complexes," (1995) *J. Am. Chem. Soc.*,117: 8933-8938. ). After hybridization, DNA duplexes are probed electrochemically in the presence or absence of a non-intercalative, redox-active moiety (See for example, US patent No 6,221,586 and Meade, T. J. and Kayyem, J.F., "Electron Transfer through DNA: Site-Specific Modification of Duplex DNA with Ruthenium Donors and Acceptors," (1995) *Angew. Chem. Int. Ed. Engl*., 34: 352-354). Interruptions in DNA-mediated electron-transfer caused by base-stacking perturbations, such as mutations, are reflected in a difference in electrical current, charge and/or potential. In one embodiment of the invention, electrochemical detection may be used to detect hybridization and to localize genetic point mutations and other base-stacking perturbations within oligonucleotide duplexes adsorbed onto electrodes.

Signal from hybridization on an array in a genotyping analysis may also be detected using invasive cleavage of oligonucleotide probes, see U.S. Patent No. 6,348,314, which is incorporated herein by reference.

In one embodiment the assay is integrated into a system that allows a crude sample to be process in an automated manner. Robots, microtitre plates, and multichannel pipettes, for example, may be used for automation. Computers and software may be used to track and manage samples. Barcodes may be used to track samples.

### Methods of use

The methods of the presently claimed invention can be used for a wide variety of applications including, for example, linkage and association studies, identification of candidate gene regions, genotyping clinical populations, correlation of genotype information to phenotype information, loss of heterozygosity analysis, and identification of the source of an organism or sample, or the population from which an organism or sample originates. Any analysis of genomic DNA may be benefited by a reproducible method of complexity management. Furthermore, the methods and enriched fragments of the presently claimed invention are particularly well suited for study and characterization of extremely large regions of genomic DNA.

### SNP discovery

In one embodiment, the methods of the presently claimed invention are used for SNP discovery and to genotype individuals. For example, any of the procedures described above, alone or in combination, could be used to identify the SNPs present in one or more specific regions of genomic DNA. Selection probes could be designed and manufactured to be used in combination with the methods of the invention to amplify only those fragments containing regions of interest, for example a region known to contain a SNP. Arrays could be designed and manufactured on a large scale basis to interrogate only those fragments containing the regions of interest. Thereafter, a sample from one or more individuals would be obtained and prepared using the same techniques which were used to prepare the selection probes or to design the array. Each sample can then be hybridized to an array and the hybridization pattern can be analyzed to determine the genotype of each individual or a population of individuals. Methods of use for polymorphisms and SNP discovery can be found in, for example, U.S. Patent No. 6,361,947 which is herein incorporated by reference in its entirety for all purposes.

In one embodiment predictions are made about the age of a polymorphism in a population. Anthropologic studies may be done using the genotype information obtained according to the methods disclosed and used to determine the age of a SNP and other anthropologic information about a SNP, for example, if a SNP originated in a specific population or geographic location.

In another embodiment one or more SNPs from two or more species are compared to identify similarities or differences between genomes. In another embodiment SNPs from two or more species are compared to determine the age of one or more SNPs.

### Correlation of Polymorphisms with Phenotypic Traits

Most human sequence variation is attributable to or correlated with SNPs, with the rest attributable to insertions or deletions of one or more bases, repeat length polymorphisms and rearrangements. On average, SNPs occur every 1,000-2,000 bases when two human chromosomes are compared. (*See*, The International SNP Map Working Group, *Nature* 409: 928-933 (2001) incorporated herein by reference in its entirety for all purposes.) Human diversity is limited not only by the number of SNPs occurring in the genome but further by the observation that specific combinations of alleles are found at closely linked sites.

Correlation of individual polymorphisms or groups of polymorphisms with phenotypic characteristics is a valuable tool in the effort to identify DNA variation that contributes to population variation in phenotypic traits. Phenotypic traits include physical characteristics, risk for disease, and response to the environment. Polymorphisms that correlate with disease are particularly interesting because they represent mechanisms to accurately diagnose disease and targets for drug treatment. Hundreds of human diseases have already been correlated with individual polymorphisms but there are many diseases that are known to have an, as yet unidentified, genetic component and many diseases for which a component is or may be genetic.

Many diseases may correlate with multiple genetic changes making identification of the polymorphisms associated with a given disease more difficult. One approach to overcome this difficulty is to systematically explore the limited set of common gene variants for association with disease.

To identify correlation between one or more alleles and one or more phenotypic traits, individuals are tested for the presence or absence of polymorphic markers or marker sets and for the phenotypic trait or traits of interest. The presence or absence of a set of polymorphisms is compared for individuals who exhibit a particular trait and individuals who exhibit lack of the particular trait to determine if the presence or absence of a particular allele is associated with the trait of interest. For example, it might be found that the presence of allele A1 at polymorphism A correlates with heart disease. As an example of a correlation between a phenotypic trait and more than one polymorphism, it might be found that allele A1 at polymorphism A and allele B 1 at polymorphism B correlate with a phenotypic trait of interest.

### Diagnosis of Disease and Predisposition to Disease

Markers or groups of markers that correlate with the symptoms or occurrence of disease can be used to diagnose disease or predisposition to disease without regard to phenotypic manifestation. To diagnose disease or predisposition to disease, individuals are tested for the presence or absence of polymorphic markers or marker sets that correlate with one or more diseases. If, for example, the presence of allele A1 at polymorphism A correlates with coronary artery disease then individuals with allele A1 at polymorphism A may be at an increased risk for the condition.

Individuals can be tested before symptoms of the disease develop. Infants, for example, can be tested for genetic diseases such as phenylketonuria at birth. Individuals of any age could be tested to determine risk profiles for the occurrence of future disease. Often early diagnosis can lead to more effective treatment and prevention of disease through dietary, behavior or pharmaceutical interventions. Individuals can also be tested to determine carrier status for genetic disorders. Potential parents can use this information to make family planning decisions.

Individuals who develop symptoms of disease that are consistent with more than one diagnosis can be tested to make a more accurate diagnosis. If, for example, symptom S is consistent with diseases X, Y or Z but allele A1 at polymorphism A correlates with disease X but not with diseases Y or Z an individual with symptom S is tested for the presence or absence of allele A1 at polymorphism A. Presence of allele A1 at polymorphism A is consistent with a diagnosis of disease X. Genetic expression information discovered through the use of arrays has been used to determine the specific type of cancer a particular patient has. (*See*, Golub et al. *Science* 286: 531-537 (2001), Yeoh et al., *Cancer Cell* 1:133-143 (2002) and Armstrong et al., *Nature Genetics* 30:41-47 (2002) hereby incorporated by reference in its entirety for all purposes.).

### Pharmacogenomics

Pharmacogenomics refers to the study of how genes affect response to drugs. There is great heterogeneity in the way individuals respond to medications, in terms of both host toxicity and treatment efficacy. There are many causes of this variability, including: severity of the disease being treated; drug interactions; and the individuals age and nutritional status. Despite the importance of these clinical variables, inherited differences in the form of genetic polymorphisms can have an even greater influence on the efficacy and toxicity of medications. Genetic polymorphisms in drug-metabolizing enzymes, transporters, receptors, and other drug targets have been linked to interindividual differences in the efficacy and toxicity of many medications. (*See*, Evans and Relling, *Science* 286: 487-491 (2001) which is herein incorporated by reference for all purposes).

An individual patient has an inherited ability to metabolize, eliminate and respond to specific drugs. Correlation of polymorphisms with pharmacogenomic traits identifies those polymorphisms that impact drug toxicity and treatment efficacy. This information can be used by doctors to determine what course of medicine is best for a particular patient and by pharmaceutical companies to develop new drugs that target a particular disease or particular individuals within the population, while decreasing the likelihood of adverse affects. Drugs can be targeted to groups of individuals who carry a specific allele or group of alleles. For example, individuals who carry allele A1 at polymorphism A may respond best to medication X while individuals who carry allele A2 respond best to medication Y. A trait may be the result of a single polymorphism but will often be determined by the interplay of several genes (See Oestreicher et al., *Pharmacogenomics J*., 1:272-287 (2001) which is herein incorporated by reference for all purposes).

In one embodiment the genotyping methods disclosed are used to identify genes that may be potential drug targets. Linkage studies to identify polymorphisms that are associated with a disease may be done. Genes that carry SNPs that are linked to a disease are candidates for drug targets. The gene product may play a role in the disease and a therapy may be designed around the role of that gene in the disease. The gene may also be identified as a research target to better understand diseases. Correlating the genotype information obtained by the methods disclosed with disease phenotype is contemplated.

In addition some drugs that are highly effective for a large percentage of the population, prove dangerous or even lethal for a very small percentage of the population. These drugs typically are not available to anyone. Pharmacogenomics can be used to correlate a specific genotype with an adverse drug response. If pharmaceutical companies and physicians can accurately identify those patients who would suffer adverse responses to a particular drug, the drug can be made available on a limited basis to those who would benefit from the drug.

Similarly, some medications may be highly effective for only a very small percentage of the population while proving only slightly effective or even ineffective to a large percentage of patients (See Yeoh et al., *Cancer Cell.,* 1:133-143 (2002) which is herein incorporated by reference for all purposes.). Pharmacogenomics allows pharamaceutical companies to predict which patients would be the ideal candidate for a particular drug, thereby dramatically reducing failure rates and providing greater incentive to companies to continue to conduct research into those drugs.

### Determination of Relatedness

There are many circumstances where relatedness between individuals is the subject of genotype analysis and the present invention can be applied to these procedures.
Paternity testing is commonly used to establish a biological relationship between a child and the putative father of that child. Genetic material from the child can be analyzed for occurrence of polymorphisms and compared to a similar analysis of the putative father's genetic material. If the set of polymorphisms in the child attributable to the father does not match the putative father, it can be concluded that the putative father is not the biological father. Determination of relatedness is not limited to the relationship between father and child but can also be done to determine the relatedness between mother and child, (see e.g. Staub et al., U.S. Pat. No. 6,187,540) or more broadly, to determine how related one individual is to another, for example, between races or species or between individuals from geographically separated populations, (see for example H. Kaessmann, et al. *Nature Genet.* 22, 78 (1999)).

### Forensics

The capacity to identify a distinguishing or unique set of forensic markers in an individual is useful for forensic analysis. For example, one can determine whether a blood sample from a suspect matches a blood or other tissue sample from a crime scene by determining whether the set of polymorphic forms occupying selected polymorphic sites is the same in the suspect and the sample. See generally, National research Council, The Evaluation of Forensic DNA evidence (Eds. Pollard et al., National Academy Press, DC, 1996). The more sites that are analyzed the lower the probability that the set of polymorphic forms in one individual is the same as that in an unrelated individual. If the set of polymorphic markers does not match between a suspect and a sample, it can be concluded (barring experimental error) that the suspect was not the source of the sample. If the set of markers does match, one can conclude that the DNA from the suspect is consistent with that found at the crime scene. If frequencies of the polymorphic forms at the loci tested have been determined (e.g., by analysis of a suitable population of individuals), one can perform a statistical analysis to determine the probability that a match of suspect and crime scene sample would occur by chance. A similar comparison of markers can be used to identify an individual's remains. For example the U.S. armed forces collect and archive a tissue sample for each service member. If unidentified human remains are suspected to be those of an individual a sample from the remains can be analyzed for markers and compared to the markers present in the tissue sample initially collected from that individual.

### Marker Assisted Breeding

Genetic markers can assist breeders in the understanding, selecting and managing of the genetic complexity of animals and plants. Agriculture industry, for example, has a great deal of incentive to try to produce crops with desirable traits (high yield, disease resistance, taste, smell, color, texture, etc.) as consumer demand increases and expectations change. However, many traits, even when the molecular mechanisms are known, are too difficult or costly to monitor during production. Readily detectable polymorphisms which are in close physical proximity to the desired genes can be used as a proxy to determine whether the desired trait is present or not in a particular organism. This provides for an efficient screening tool which can accelerate the selective breeding process.

The methods of complexity reduction and analysis of reduced complexity samples disclosed may be useful for genotyping organisms that are useful as agricultural products or as model organisms for research, such as yeast, drosophila and Arabidopsis.Genotypes may be used for determination of origin, selection of desired traits, prediction of phenotype, for example.

### Clinical trial

In one embodiment genotypes are used to pre-select individuals for clinical studies. For a particular clinical study, for example a clinical trial for a drug, it may be desirable to select patients based on common genotype. In another embodiment patients are selected on the basis of varied genotype. Different clinical groups may be defined by genotype. In another embodiment genotyping information is used to define group characteristics in a clinical setting. For example, a study may identify one group of individuals that responds positively to a treatment regime and a second group that responds poorly to the treatment or not at all. Genotype information from the groups may be associated with drug treatment response. This information may then be used to predict how a patient will respond to the drug treatment. One or more genotypes may be associated with a favorable response to the treatment and another genotype may be associated with an unfavorable response to the treatment. Treatment options can be selected based on genotype.

### Expression analysis

In another embodiment genotypes are linked to gene expression profiles. The presence of certain alleles of one or more polymorphism may impact the expression of the gene containing the polymorphism or the expression of other genes in the organism. Genotype and gene expression may be correlated using the methods disclosed to identify and analyze correlation. Methods are provided for identification of genes that are imprinted or genes that show allelic imbalance in expression pattern. The expression products transcribed from genes that are present in the genome as two or more alleles may be distinguished by hybridization to an array designed to interrogate individual alleles. Genes whose transcription products are present in amounts that vary from expected are candidates for allelic imbalance, imprinting and imprinting errors. In another embodiment gene deletions are analyzed.

In another embodiment sites of methylation are determined. Methylation of CpG dinucleotides is an important regulator of gene expression in mammals. The methods disclosed may be used to rapidly analyze many possible sites of methylation in a genome in parallel. In one embodiment the methylation status of a cytosine is analyzed using restriction digestion with two restriction enzymes that recognize the same recognition site but are differentially sensitive to methylation. In one embodiment *Hpa*II and *Msp*I are used and the cytosine is part of a CpG dinucleotide. *Hpa*II and *Msp*I are isoschizomers which cleave at recognition site CCGG (see, New England Biolab Catalogue, which is incorporated herein by reference in its entirety). Cleavage by *Hpa*II is blocked by methylation while *Msp*I cleaves independent of methylation. A genomic DNA sample is digested with a restriction enzyme and adapters are ligated to the fragments to generate a population of adapter-modified fragments. The sample is divided into three fractions. One fraction is fragmented with *Hpa* II, a second fraction is fragmented with *Msp*I and the final fraction is left untreated. Each of the fractions is then amplified using primers to the adapters. The amplified products are then hybridized to an array of probes designed to interrogate the presence or absence of specific fragments, for example, the array disclosed in US patent application nos. 10/264,945, 09/916,135 and 60/417,190 each of which is incorporated herein by reference. Fragments that have the CCGG recognition site will either be cleaved in both the *Msp*I and *Hpa*II fractions if the CpG is unmethylated or will be cleaved in the *Msp*I fraction but not the *Hpa*II fraction if the CpG is methylated. After cleavage the samples are amplified using primers to the adapter sequences. If a fragment has been cleaved by *Msp*I or *Hpa*II the fragment will not be amplified in the PCR reaction because the resulting fragments will have the adapter sequence, and therefore the priming site, only on one end.

In another embodiment sites of transcription factor binding are mapped by cross linking the transcription factor to nucleic acid, immunoprecipitation of the cross linked transcription factors using antibodies that recognize the transcription factor and then analysis of the cross linked nucleic acid using one or more of the SNP detection methods disclosed above. In one embodiment the nucleic acid associated with the transcription factor or factors is analyzed by hybridization to an array designed to interrogate SNPs. One allele of a gene may preferentially associate with a transcription factor while a second allele associates poorly. Differences in allelic association may be detected by the methods of complexity reduction and genotyping disclosed herein.

### EXAMPLES

### Example 1

Digestion: Digest 300 ng human genomic in a 20 µl reaction in 1X NEB buffer 2 with 1X BSA and 1U/µl *Xba*1 (NEB). Incubate the reaction at 37°C overnight or for 16 hours. Heat inactivate the enzyme at 70°C for 20 minutes.

Ligation: Mix the 20 µl digested DNA with 1.25 µl of 5 µM adaptor, 2.5 µl 10X ligation buffer and 1.25 µl 400U/µl ligase. The final concentrations are 12 ng/µl DNA, 0.25 µM adaptor, 1X buffer and 20U/µl ligase. Incubate at 16°C overnight. Heat inactivate enzyme at 70°C for 20 minutes. Sample may be stored at -20°C.

Amplification: Mix the 25µl ligation reaction in a 1000ul PCR reaction. Final concentrations of reagents are as follows: 1X PCR buffer, 250µM dNTPs, 2.5mM MgCl₂, 0.5µM primer, 0.3ng/µl ligated DNA, and 0.1U/µl Taq Gold. The reaction is divided into 10 tubes of 100µl each prior to PCR.

Reaction cycles are as follows: 95°C for 10 minutes; 20 cycles of 95° for 20 seconds, 58°C for 15 seconds and 72°C for 15 seconds; and 25 cycles of 95°C for 20 seconds, 55°C for 15 seconds, and 72°C for 15 seconds followed by an incubation at 72°C for 7 minutes and then incubation at 4°C indefinitely. Following amplification 3 µl of the sample may be run on a 2% TBE minigel at 100V for 1 hour.

Fragmentation and Labeling: PCR reactions were cleaned and concentrated using a Qiagen PCR clean up kit according to the manufacturer's instructions. Eluates were combined to obtain a sample with approximately 20 µg DNA, approximately 250-300 µl of the PCR reaction was used. The 20 µg product should be in a volume of 43 µl, if necessary vacuum concentration may be required. The DNA in 43µl was combined with 5µl 10X NEB buffer 4, and 2µl 0.09U/µl DNase and incubated at 37°C for 30 min, 95°C for 10 minutes then to 4°C. DNA was labeled with TdT under standard conditions.

Hybridization: Standard procedures were used for hybridization, washing, scanning and data analysis. Hybridization was to an array designed to detect the presence or absence of a collection of human SNPs present on *Xba*I fragments of 400 to 1,000 base pairs.

### Example 2

Genomic DNA was digested with *Xba*I by mixing 5 µl 50 ng/µl human genomic DNA (Coriell Cell Repositories) with 10.5 µl H₂O (Accugene), 2 µl 10X RE buffer 2 (NEB, Beverly, MA), 2 µl 10X BSA (NEB, Beverly, MA), and 0.5 µl *Xba*I (NEB, Beverly, MA). The reaction was incubated at 30°C for 2 hours, then the enzyme was inactivated by incubation at 70°C for 20 min and then to 4°C. The reaction may be stored at -20°C.

For ligation of the adapters the digested DNA was then mixed with 1.25 µl 5uM adaptor in TE pH 8.0, 2.5 µl T4 DNA ligation buffer and 1.25 µl T4 DNA Ligase (NEB, Beverly, MA) which is added last. The reaction was incubated at 16°C for 2 hours then at 70°C for 20 min and then to 4°C. The 25 µl ligation mixture is then diluted with 75 µl H₂0 and may be stored at -20°C.

For PCR 10 µl of the diluted ligated DNA is mixed with 10 µl PCR buffer II (Perkin Elmer, Boston, MA), 10 µl 2.5 mM dNTP (PanVera Takara, Madison, WI), 10 µl 25 mM MgCl₂, 7.5 µl 10 µM primer (for a final concentration of 0.75 µM), 2 µl 5U/µl Taq Gold (Perkin Elmer, Boston, MA) and 50.5 µl H₂O. For each array four 100 µl reactions were prepared. Amplification was done using the following program: 95°C for 3 min; 35 cycles of 95°C for 20 sec, 59°C for 15 sec and 72°C for 15 sec; and a final incubation at 72°C for 7 min. The reactions were then held at 4°C. The lid heating option was selected.

The PCR reactions were then purified by mixing the 100 µl PCR reaction with 500 µl PB or PM buffer into Qiagen columns (Valencia, CA) and the column was centrifuged at 13,000 rpm for 1 min. Flow through was discarded and 750 µl PE buffer with ethanol was added into the column to wash the sample and the column was spun at 13,000 rpm for 1 min. The flow through was discarded and the column was spun at 13,000 rpm for another 1 min. The flow through was discarded and the column was placed in a new collection tube. For 2 of the 4 samples 30 µl of EB elution buffer pH 8.5 was added to the center of the QIAquick membrane to elute the sample and the columns were allowed to stand at room temperature for 5 min and then centrifuged at 13,000 for 1 min. The elution buffer from the first 2 samples was then used to elute the other 2 samples and the eluates were combined. The DNA was quantified and diluted so that 48 µl contains 20 µg DNA.

The DNA was fragmented by mixing 48 µl DNA (20 µg), 5 µl RE Buffer 4, and 2 µl 0.09 U/ µl DNase in a total volume of 55 µl. The reaction was incubated at 37°C for 30 min then 95°C for 15 min and then held at 4°C.

Fragments were labeled by incubating 50 µl fragmented DNA, 13 µl 5X TdT buffer (Promega, Madison, WI), 1 µl 1 mM biotinolated-ddATP (NEN Life Sciences, Boston, MA), and 1 µl TdT (Promega, Madison, WI) at 37°C overnight then at 95°C for 10 min, then held at 4°C.

Hybridization mix is 12 µl 1.22 M MES, 13 µl DMSO, 13 µl 50X Denharts, 3 µl 0.5M EDTA, 3 µl 10 mg/ml herring sperm DNA, 3 µl 10nM oligo B2, 3 µl 1 mg/ml Human Cot-1, 3 µl 1% Tween-20, and 140 µl 5M TMACL. 70 µl labeled DNA was mixed with 190 µl hybridization mix. The mixture was incubated at 95°C for 10 min, spun briefly and held at 47.5°C. 200 µl of the denatured mixture was hybridized to an array at 47.5°C for 16 to 18 hours at 60 rpm.

Staining mix was 990 µl H₂0, 450 µl 20X SSPE, 15 µl Tween-20, 30 µl 50% Denharts. For the first stain mix 495 µl staining mix with 5 µl 1 mg/ml streptavidin (Pierce Scientific, Rockford, IL), for the second stain mix 495 µl staining mix with 5 µl 0.5 mg/ml biotinylated anti-streptavidin antibody (Vector Labs, Burlingame, CA) and for the third stain mix 495 µl staining mix with 5 µl 1 mg/ml streptavidin, R-phycoerythrin conjugate (Molecular Probes, Eugene, OR). Wash and stain under standard conditions.

### CONCLUSION

From the foregoing it can be seen that the present invention provides a flexible and scalable method for analyzing complex samples of DNA, such as genomic DNA. These methods are not limited to any particular type of nucleic acid sample: plant, bacterial, animal (including human) total genome DNA, RNA, cDNA and the like may be analyzed using some or all of the methods disclosed in this invention. This invention provides a powerful tool for analysis of complex nucleic acid samples. From experiment design to isolation of desired fragments and hybridization to an appropriate array, the above invention provides for fast, efficient and inexpensive methods of complex nucleic acid analysis.

All publications and patent applications cited above are incorporated by reference in their entirety for all purposes to the same extent as if each individual publication or patent application were specifically and individually indicated to be so incorporated by reference. Although the present invention has been described in some detail by way of illustration and example for purposes of clarity and understanding, it will be apparent that certain changes and modifications may be practiced within the scope of the appended claims.

## Claims

1. A method of reducing the complexity of a first nucleic acid sample to generate a reduced complexity sample comprising a plurality of target sequences wherein the steps of complexity reduction comprise:
(i) reducing the complexity of a first nucleic acid sample to generate a second nucleic acid sample in a first complexity reduction step wherein complexity is reduced based on a physical or chemical property of the sequences in the first nucleic acid sample; and
(ii) reducing the complexity of the second nucleic acid sample to generate the reduced complexity sample in a second complexity reduction step comprising:
(1) fragmenting said second nucleic acid sample to produce sample fragments;
(2) ligating at least one adaptor to the sample fragments; and
(3) generating the reduced complexity sample by amplifying a subset of sample fragments wherein the plurality of target sequences is enriched in the reduced complexity sample.

2. The method of claim 1 wherein the first complexity reduction step comprises removal of repetitive sequences.

3. The method of claim 2 wherein repetitive sequences are removed by incubating the first nucleic acid sample with Cot-1 DNA and removing the Cot-1 DNA and Cot-1 DNA complexes.

4. The method of claim 1 wherein the first complexity reduction step comprises isolating active chromatin from the first nucleic acid sample wherein the second nucleic acid sample comprises the nucleic acids present in the isolated active chromatin.

5. The method of claim 4 wherein active chromatin is isolated by a method comprising immunoprecipitation of acetylated histones with an antibody.

6. The method of claim 1 wherein the first complexity reduction step is isolating one or more individual chromosomes or chromosome fragments from the first nucleic acid sample by pulsed field gradient gel electrophoresis.

7. The method of claim 1 wherein the first complexity reduction step comprises isolating one or more individual chromosomes from the first nucleic acid sample by affinity chromatography.

8. The method of claim 7 wherein a solid support is used to isolate individual chromosomes and the solid support is an array, a nylon or nitrocellulose membrane, a resin or a bead.

9. The method of claim 1 wherein the first complexity reduction step is isolating nucleic acids from a somatic cell hybrid containing a subset of chromosomes from an organism.

10. The method of claim 9 wherein the somatic cell hybrid contains 1 to 15 human chromosomes.

11. The method of claim 9 wherein the somatic cell hybrid contains fragments of one or more human chromosomes.

12. The method of claim 1 wherein amplification is by PCR using a primer that is complementary to the adaptor.

13. The method of claim 1 wherein the step of fragmenting said first nucleic acid sample comprises digestion with at least one restriction enzyme.

14. The method of claim 1 wherein the sequences that are enriched in the reduced complexity sample comprise at least 0.1% of said first nucleic acid sample.

15. The method of claim 1 wherein the sequences enriched in the reduced complexity sample comprise at least 10% of said first nucleic acid sample.

16. The method of claim 1 wherein said first nucleic acid sample is genomic DNA, DNA, cDNA derived from RNA or cDNA derived from mRNA.

17. The method of claim 1 wherein the target sequences are 5000 base pairs long or less.

18. The method of claim 1 wherein the subset of sample fragments is comprised of fragments that are about 2000 base pairs long or less.

19. The method of claim 1 wherein the subset of sample fragments is comprised of fragments that are about 5000 base pairs long or less.

20. The method of claim 1 wherein said fragmenting, ligating and amplifying steps are performed in a single tube.

21. The method of claim 1 wherein said target sequences comprise sequence variations.

22. The method of claim 21 wherein said sequences variations are single nucleotide polymorphisms.

23. The method of claim 22 wherein at least one of the single nucleotide polymorphisms is associated with a phenotype.

24. The method of claim 22 wherein at least one of the single nucleotide polymorphisms is associated with the efficacy of a drug.

25. The method of claim 22 wherein at least one of the single nucleotide polymorphisms is associated with a haplotype.

26. A method for genotyping a first nucleic acid sample comprising reducing the complexity of the first nucleic acid sample according to the method of claim 1 and detecting the identity of one or more alleles of one or more polymorphisms present in said collection of target sequences.

27. The method of claim 26 wherein the step of detecting the identity of the one or more alleles comprises detecting hybridization of a molecular beacon probe, using electrical pore analysis, electrical conductance analysis, atomic force microscopy analysis, pyrosequencing analysis, MALDI-TOF mass spectrometry analysis, Surface Enhanced Raman Scattering analysis, current amplitude analysis, eSensor system analysis, or electrochemical DNA biosensor analysis.

28. A method for analyzing a first nucleic acid sample comprising:
reducing the complexity of the first nucleic acid sample according to the method of claim 1;
providing a nucleic acid array that is designed to be complementary to sequences in the plurality of target sequences;
hybridizing the reduced complexity sample to the array;
generating a hybridization pattern resulting from the hybridization; and
analyzing the hybridization pattern.

29. The method of claim 28 wherein the nucleic acid array comprises probes to genotype at least one single nucleotide polymorphism in the plurality of target sequences.

30. The method of claim 28 wherein the step of analyzing said hybridization pattern determines the presence or absence of DNA sequence variation in the first nucleic acid sample.

31. The method of claim 28 further comprising using a computer to predict sequences that will be enriched in the third nucleic acid sample.
